(19) Europäisches Patentamt
European Patent Office
Office européen des brevets

(11) Publication number: **0 638 588 A1**

(12) **EUROPEAN PATENT APPLICATION**

(21) Application number: **94111469.6**

(22) Date of filing: **22.07.94**

(51) Int. Cl.$^6$: **C07K 5/06**, C07K 5/08, C07K 5/10, C07K 7/06

(30) Priority: **22.07.93 JP 181735/93**
**25.01.94 JP 6670/94**

(43) Date of publication of application:
**15.02.95 Bulletin 95/07**

(84) Designated Contracting States:
**AT BE CH DE DK ES FR GB GR IE IT LI LU NL PT SE**

(71) Applicant: **TAKEDA CHEMICAL INDUSTRIES, LTD.**
**3-6, Doshomachi 2-chome**
**Chuo-ku,**
**Osaka 541 (JP)**

(72) Inventor: **Hida, Tsuneaki**
**Takeda Kasugahaitsu, 7-9,**
**Kasuga, 1-chome**
**Tsukuba,**
**Ibaraki 305 (JP)**
Inventor: **Tanida, Seiichi**
**Takadainishi**
**Nagaokakyo, Kyoto 617 (JP)**
Inventor: **Harada, Setsuo**
**Seiwadainishi 2-chome**
**Kawanishi,**
**Hyogo 666-01 (JP)**
Inventor: **Yukishige, Koichi**
**10-1-514, Makamicho 6-chome**
**Takatsuki,**
**Osaka 569 (JP)**

(74) Representative: **Casalonga, Axel**
**BUREAU D.A. CASALONGA - JOSSE**
**8, avenue Percier**
**F-75008 Paris (FR)**

(54) **Derivatives of 6,7-Dihydroxy-4-thiaheptonic acid and their use.**

(57) A compound of the formula:

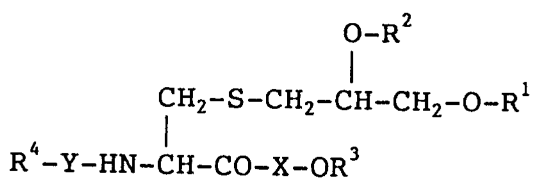

wherein each of $R^1$ and $R^2$ is hydrogen or an aliphatic acyl group, each of $R^3$ and $R^4$ is hydrogen or a protecting group, X is an amino acid sequence consisting of 1 to 10 amino acid residues which may be protected, Y is an amino acid sequence consisting of 1 to 5 amino acids residues which may be protected, bounding at a terminal carboxyl group, or a salt thereof has an activity of remarkably improving hematopoietic disorder and is useful as an immuno-stimulating agent or an agent for treating thrombocytopenia.

EP 0 638 588 A1

FIELD OF THE INVENTION

This invention relates to derivatives of 6,7-dihydroxy-4-thiaheptanoic acid, which are useful as therapeutic agent of leukocytopenia or thrombocytopenia caused by various resons, diseases due to a decrease of leukocyte or thrombocyte, diseases requiring, from the therapeutic viewpoint, increase of bone marrow cells, leukocytes or thrombocytes.

BACKGROUND OF THE INVENTION

In Hoppe-Seyler's Zeitschrift fur Physiologische Chemie) 364, 593-606 (1983), a lipoprotein peptide of the E. coli origin, which is shown by the formula:

```
                        O-COC15H31
          3   4 5       |
          CH2-S-CH2-CH-CH2-O-COC15H31
          |             6   7
C15H31CONHCH-CO-Ser-Ser-Asn-Ala-OH
          2   1
```

is disclosed.

And, in JPA H4(1992)-46194, WS 1279A substance shown by the formula:

```
                    O-COC15H31
                    |
          CH2-S-CH2-CH-CH2-O-COC15H31
          |
C15H31CONHCH-CO-Asn-Ser-Gly-Gly-Ser-OH
```

is disclosed.

Optical active compounds of these are described in JP-AH4(1992)-099796; Chem. Pharm. Bull. 39, 2590-2596 (1991) and Peptide Chemistry, 29, 361-366 (1991).]

However, these 4-thiaheptanoic acid compounds have $C_{15}H_{31}CO$- substituted amino at the 2-position. On the other hand, the compounds of the present invention have amino acid sequence-substituted amino at the 2-position. Thus the compounds of the present invention are structurally different from prior art compounds as mentioned above. Moreover, the prior art references do not describe the therapeutic action of thrombocytopenia.

Abbreviations of amino acid, peptide or the like used in the present specification are based on those in according with IUPAC-IUB Commission on Biochemical Nomenclature or those conventionally used in the relevant fields, and possible optical isomers of amino acid are L-isomers, unless otherwise specified.

Chemotherapy or radiotherapy of cancers causes serious leukocytopenia or serious thromobocytopenia. The former induces lowering of resistance against infections or other various diseases so that sufficient therapeutic effects are not expected. The latter induces insufficiency of hemostatic mechanism so that sufficient therapeutic effects are not expected. These are being taken up as a grave concern in the field of cancer therapy. Under such circumstances, development of drugs, which mitigate the suppression of hematopoietic function caused by these therapeutic methods and are capable of promoting the recovery of leukocyte number or thrombocyte number, has been ardently desired. Further, in the therapy by bone marrow transplantation, drugs capable of promoting the proliferation of bone marrow cells then transplanted and capable of recovering the number of leukocyte promptly are desired. Furthermore, these drugs can be used for therapeutic agents of thrombocytopenia after bone marrow transplantation or autoimmune diseases accompanied by thrombocytopenia, such as aplastic anemia and paroxymal thrombocytopenic purpura and so on.

While taking the present circumstances mentioned above into consideration, the present inventors pursued their studies, from a fresh viewpoint, on compounds having the action of increasing the number of leukocyte and thrombocyte. As the result, the present inventors found that the novel 6,7-dihydroxy-4-thiaheptanoic acid derivatives promote the proliferation of bone marrow cells of mice and increase the number of peripheral leukocytes. Further, said compounds also stimulate bone marrow cells of mice so that promote proliferation and differentiation of megakaryocytes. Based on these findings, the present inventors made further studies to complete the present invention.

## SUMMARY OF THE INVENTION

This invention provides:

1) A compound of the formula (I)

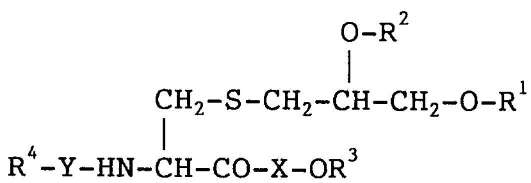

(I)

wherein each of $R^1$ and $R^2$ is hydrogen or an aliphatic acyl group, each of $R^3$ and $R^4$ is hydrogen or a protecting group, X is an amino acid sequence consisting of 1 to 10 amino acid residues which may be protected, Y is an amino acid sequence consisting of 1 to 5 amino acids residues which may be protected, bounding at a terminal carboxyl group, or a salt thereof;

2) A compound according to 1), wherein the aliphatic acyl group is a $C_{2-30}$ aliphatic acyl group;

3) A compound according to 1), wherein at least one of $R^1$ and $R^2$ is an aliphatic acyl group;

4) A compound according to 1), wherein X is an amino acid sequence consisting of 1 to 10 amino acid residues which may be protected, containing at least an amino acid residue which have a water-solubility enhancing group;

5) A compound according to 4), wherein the amino acid residue having a water-solubility enhancing group is an acidic amino acid residue;

6) A compound according to 4), wherein the amino acid residue having a water solubility enhancing group is a basic amino acid residue;

7) A compound according to 1), wherein at least one of $R^1$ and $R^2$ is a $C_{2-18}$ aliphatic acyl group, X is an amino acid sequence consisting of 2 to 5 optionally protected amino acid residues being selected from the group consisting of glutamyl, D-glutamyl, glycyl and lysysl, Y is an amino acid sequence consisting of 1 to 4 optionally protected amino acid residues being selected from the group consisting of glutamyl, glycyl, tryosyl, lysyl and $\beta$-alanyl;

8) A compound according to 1), wherein the compound is (2R,6R)-2-glycyl-amino-6,7-bis-(hexadecanoyloxy)-4-thiaheptanoyl-glutamyl-glycyl-D-glutamic acid;

9) A compound according to 1), wherein the compound is (2R,6R)-2-glutamyl-glycyl-amino-6,7-bis-(hexadecanoyloxy)-4-thiaheptanoyl-glutamyl-glycyl-D-glutamic acid;

10) A compound according to 1), wherein the compound is (2R,6R)-2-tyrosyl-amino-6,7-bis-(hexadecanoyloxy)-4-thiaheptanoyl-glutamyl-glycyl-D-glutamic acid;

11) A compound according to 1), wherein the compound is (2R,6R)-2-lysyl-amino-6,7-bis-(hexadecanoyloxy)-4-thiaheptanoyl-glutamyl-glycyl-D-glutamic acid;

12) A compound according to 1), wherein the compound is (2R,6R)-2-glycyl-amino-6,7-bis-(hexadecanoyloxy)-4-thiaheptanoyl-glycyl-glutamyl-glutamic acid;

13) An immuno-stimulating composition having a leukocyte-increasing action, which comprises a compound or a salt thereof as defined in 1); and

14) A composition for treating thrombocytopenia, which comprises a compound or a salt thereof as defined in 1).

## DETAILED DESCRIPTION OF THE INVENTION

Referring to the formula (I), examples of the aliphatic acyl group shown by $R^1$ or $R^2$ include aliphatic acyl groups derived from aliphatic carboxylic acids.

Examples of the aliphatic acyl groups include $C_{1-34}$ aliphatic acyl groups, for example, $C_{1-34}$ saturated aliphatic acyl groups (e.g. formyl, acetyl, propionyl, butyryl, isobutyryl, valeryl, isovaleryl, octanoyl, decanoyl, dodecanoyl, tridecanoyl, tetradecanoyl, pentadecanoyl, hexadecanoyl, heptadecanoyl, octadecanoyl,

nonadecanoyl, nocosanoyl, tetracosanoyl, hexacosanoyl, ethyldodecanoyl, methyltridecanoyl, ethyltridecanoyl, methyltetradecanoyl, ethyltetradecanoyl, methylpentadecanoyl, ethylpentadecanoyl, methylhexadecanoyl, ethylhexadecanoyl, methylheptadecanoyl, ethylheptadecanoyl, methyloctadecanoyl, ethyloctadecanoyl, octacosanoyl, triacontanoyl, dotriacontanoyl, tetratriacontanoyl and so on, $C_{3-24}$ unsaturated aliphatic acyl groups (e.g. acryloyl, propioloyl, methacryloyl, crotonoyl, isocrotonoyl, myristoleoyl, oleoyl, palmitoleoyl, elaidoyl, cis,cis-9,12-octadecadienoyl, 9,12,15-octadecatrienoyl, 9,11,13-octadecatrienoyl, 5,8,11,14-eicosatetraenoyl, cis-15-tetracosaenoyl and so on).

Preferable examples of aliphatic acyl groups include $C_{2-30}$ aliphatic acyl groups, for example, $C_{2-30}$ saturated aliphatic acyl groups (e.g. acetyl, propionyl, butyryl, isobutyryl, valeryl, isovaleryl, octanoyl, decanoyl, dodecanoyl, tetradecanoyl, hexadecanoyl, ethyldodecanoyl, methyltridecanoyl, ethyltridecanoyl, methyltetradecanoyl, ethyltetradecanoyl, methylpentadecanoyl, ethylpentadecanoyl, methylhexadecanoyl, ethylhexadecanoyl, methylheptadecanoyl, ethylheptadecanoyl, methyloctadecanoyl, ethyloctadecanoyl, octadecanoyl, nonacosanoyl, tetracosanoyl, hexacosanoyl, octacosanoyl, triacontanoyl, etc.), $C_{14-24}$ unsaturated aliphatic acyl groups (e.g. myristoleoyl, oleoyl, palmitoleoyl, elaidoyl, cis,cis-9,12-octadecadienoyl, 9,12,15-octadecatrienoyl, 9,11,13-octadecatrienoyl, 5,8,11,14-eicosatetraenoyl, cis-15-tetracosaenoyl, etc.). Especially preferable examples of the aliphatic acyl groups shown by $R^1$ or $R^2$ include $C_{2-18}$ aliphatic acyl groups, for example, $C_{10-18}$ saturated aliphatic acyl groups (e.g., decanoyl, dodecanoyl, tetradecanoyl, hexadecanoyl, octadecanyol and so on), or $C_{14-18}$ unsaturated aliphatic acyl groups (e.g., myristoleoyl, oleoyl, palmitoleoyl, cis,cis,-9,12-octadecadienyol, 9,12,15-octadecatrienoyl and so on).

Especially preferably, at least one of $R^1$ and $R^2$ is an aliphatic acyl group the aliphatic acyl groups are, for example, $C_{10-18}$ saturated aliphatic acyl groups (e.g., decanoyl, dodecanoyl, tetradecanoyl, hexadecanoyl, octadecanyol and so on), or $C_{14-18}$ unsaturated aliphatic acyl groups (e.g., myristoleoyl, oleoyl, palmitoleoyl, (cis,cis)-9,12-octadecadienyol, 9,12,15-octadecatrienoyl and so on). Among others, hexadecanoyl (palmitoyl) is most preferable.

In the formula (I), the amino acid that may be protected in the amino acid sequence shown by X means a compound having in its molecule an amino group and an acidic group (e.g. carboxyl group, sulfo group).

More concretely, there may be mentioned, for example, amino acid which constitutes protein (e.g. aliphatic monoamino monocarboxylic acid such as glycine, alanine, valine, leucine, isoleucine or the like, aliphatic hydroxyamino acid such as serine, threonine or the like, monoamino dicarboxylic acid such as aspartic acid, glutamic acid or the like, monoamino dicarboxylic acid amide such as asparagine, glutamine or the like, aromatic amino acid such as phenylalanine, tyrosine, tryptophan or the like, iminocarboxylic acid such as proline, hydroxyproline or the like, diamino monocrboxylic acid such as arginine, lysine, histidine or the like and sulfur-containing amino acid such as methionine, cystine, cysteine or the like), amino acids obtained from natural sources us, for example, metabolites of microorganisms or components of animal or plant [e.g. aliphatic monoamino monocarboxylic acid such as L-$\alpha$-aminobutyric acid, $\gamma$-aminobutyric acid, $\beta$-aminoisobutyric acid, $\beta$-alanine, homoserine, $\alpha$-methyl-D-serine, O-carbamyl-D-serine, $\delta$-hydroxy-$\gamma$-oxo-norvaline or the like, monoamino dicarboxylic acid such as L-$\alpha$-aminoadipic acid, L-$\beta$-aminoadipic acid, L-theanine, L-$\gamma$-methylene glutamic acid, L-$\gamma$-methyl glutamic acid or the like, diamino monocarboxylic acid such as L-ornithine, $\beta$-lysine, $\alpha$,$\beta$-diaminopropionic acid, L-$\alpha$,$\gamma$-diaminobutyric acid or the like, diaminodicarboxylic acid such as diaminopimelic acid or the like, sulfonic acid-containing monoamino monocarboxylic acid such as cysteic acid or the like, amino group-containing sulfonic acid such as taurine or the like, aromatic amino acid such as kynurenine, 3,4-dioxyphenyl-L-alanine or the like, heterocycle amino acid such as 2,3-dicarboxyaziridine, [S]-2-amino-3-(isoxazolin-5-on-4-yl)-propionic acid, anticapsin or the like, carboxylic acid having 2 or more amino group such as L-4-oxalysine, L-4-oxolysine, [3R,5R]-3,6-diamino-5-hydroxyhexanoic acid or the like, sulfur-containing amino acid such as lanthionine, S-methyl-L-cysteine or the like, cyclic amino acid such as pipecolic acid, azetidine-2-carboxylic acid, [1R,2S]-2-aminocyclopentane-1-carboxylic acid or the like, amino acid substituted with a specific functional group such as citrulline, alanosine, L-azaserine or the like], and amino acids obtained by organic synthesis [e.g.6-aminohexanoic acid, 8-aminooctanoic acid, 12-aminododecanoic acid, 4-aminobenzoic acid, 4-(aminomethyl)benzoic acid, 4-(N-carboxymethyl)aminomethyl)benzoic acid, etc.].

The amino acid residue in the amino acid sequence shown by X means a divalent group which is derived from amino acid and which has chemical bounds at the amino group and acidic group, respectively.

Preferably, X is an amino acid sequence consisting of 1 to 10 amino acid residues which may be protected, containing at least an amino acid residue having a hydrophilic group.

Examples of the amino acid having a hydrophilic group include the acidic amino acid and basic amino acid, preferably, the acidic amino acid.

Examples of the acidic amino acids include a compound having one or more acidic functional groups (e.g. carboxyl group, sulfo group, etc.) in addition to a carboxyl and amino group. Preferable examples of

the acidic amino acid include a compound having one amino group and two or more carboxyl groups.

More concretely, there may be mentioned, for example, amino dicarboxylic acids (e.g. aspartic acid, glutamic acid, L-α-aminoadipic acid, L-β-aminoadipic acid, 2,3-dicarboxyaziridine, etc.), more preferably, α-aminodicarboxylic acids (e.g. aspartic acid, glutamic acid, L-α-aminoadipic acid, etc.) and, among them, aspartic acid and glutamic acid are especially preferable.

Examples of the basic amino acid mentioned include a compound having one or more basic functional groups (e.g. amino, nitrogen-containing basic heterocyclic group such as imidazolyl or indolyl, or, guanidino and so on) in addition to a carbonyl and amino group. Preferable examples of the basic amino acid include a compound having one amino group, one carboxyl group and one or more basic functional groups as mentioned above.

More concretely, for example, a basic amino acid constituting protein (e.g. arginine, histidine, lysine, etc.) and a basic amino acids obtained from natural sources such as, for example, metabolites of microorganisms or components of animals and plants [e.g. diamino carboxylic acids such as L-ornithine, β-lysine, α,β-diaminopropionic acid, L-α,γ-diaminobutyric acid or the like, L-4-oxalysine, L-4-oxolysine, (3R,5R)-3,6-diamino-5-hydroxyhexanoic acid, etc.], more preferably a basic α-amino acid constituting protein (e.g. arginine, histidine, lysine, etc.) and a basic α-amino acid obtained from natural sources as, for example, metabolites of microorganisms or components of animals and plants tissues (e.g. L-ornithine, α,β-diaminopropionic acid, L-α,γ-diaminobutyric acid, L-4-oxalysine, L-4-oxolysine, etc.), and among them, lysine, arginine and histidine are especially preferable.

As the protecting group which may be present on any of the 1 - 10 amino acid residues constituting the amino acid sequence of X, use is made of protecting groups employed for protecting amino group, carboxyl group or hydroxyl group in peptide synthesis. These are protecting groups which can be removed by, for example, hydrolysis, hydrogenolysis, reduction, aminolysis or hydrazinolysis.

Examples of the amino-protecting groups include urethane-type protecting groups (e.g. benzyloxycarbonyl, t-butyloxycarbonyl, allyloxycarbonyl, t-amyloxycarbonyl, isobornyloxycarbonyl, 4-methoxybenzyloxycarbonyl, 2-chlorobenzyloxycarbonyl, adamantyloxycarbonyl, 9-fluorenylmethyloxycarbonyl, 2,2,2-trichloroethyloxycarbonyl or the like), acyl-type protective groups (e.g. $C_{1-6}$ lower fatty acid residues optionally having such a substituent as formyl, acetyl, propyl, trifluoroacetyl, chloroacetyl, or the like, phthalyl, tosyl, 2-nitrosulfenyl, 4-methoxy-2-nitrosulfenyl, benzoyl or the like), and aralkyl-type protecting groups (e.g. trityl, benzhydryl, benzyl or the like).

Among these, urethane-type protecting groups are especially preferable.

Carboxyl groups are protected by converting them into, for example, amido group, hydrazido group or ester. Preferable amido groups or hydrazido groups are those substituted with a suitable substituent. Preferable amido groups are those substituted with a $C_{7-19}$ aralkyl group optionally substituted with, for example, alkoxy group (e.g. 3,4-dimethoxybenzyl group or bis(p-methoxyphenyl)-methyl group). Preferable hydrazide group are those substituted with, for example, $C_{1-6}$ alkyloxycarbonyl group optionally substituted with halogen (e.g. fluorine, chlorine, bromine, etc.) $C_{6-12}$ aryl group (e.g. phenyl, p-biphenylyl, etc.) (e.g. benzyloxycarbonyl group, trichloroethyloxycarbonyl group, tert-butyloxycarbonyl group, 2-(p-biphenylyl)-isopropyloxycarbonyl, etc.), halogenated $C_{2-6}$ alkanoyl group (e.g. trifluoroacetyl group, etc.) and $C_{7-19}$ aralkyl group (e.g. trityl group, benzhydryl group, benzyl group, etc.), among others. Further, the carboxyl groups may be protected as esters with an optionally substituted lower alcohol (e.g. methanol, ethanol, cyanomethyl alcohol, benzoylmethyl alcohol, tert-butanol, etc.), aralkanol [e.g. benzyl alcohol or benzohydrols (e.g. benzohydrol, p-nitrobenzyl alcohol, p-methoxybenzyl alcohol, 2,4,6-trimethylbenzyl alcohol, etc.), optionally substituted with, for example, lower alkyl group, lower alkoxy group or halogen atom], phenol and thiophenol optionally substituted with an electron withdrawing group (e.g. thiophenol, thiocresol, p-nitrothiophenol, 2,4,5- or 2,4,6-trichlorophenol, p-cyanophenol, p-methanesulfonylphenol, etc.), and further, with N-hydroximide (e.g. N-hydroxysuccinimide, N-hydroxyphthalimide, etc.), N-hydroxypiperidine, 8-hydroxyquinoline or the like.

As the special protecting group of carboxyl, which can be removed under neutral conditions, mention is made of a hydrocarbyl-silyl-ethyl group, for example, 2-(trimethylsilyl)ethyl group (described in German Patent Application Laid-open No. 2,706,490).

Hydroxyl can be protected with, for example, acylation or etherification.

The especially preferable acyl group in the case of acylation is a group derived from carbonic acid (e.g. benzyloxycarbonyl group or ethyloxycarbonyl group). For etherification, benzyl group, tetrahydropyranyl group or tert-butyl group, for example, is preferable. And for the protection of hydroxyl group, 2,2,2-trifluoro-1-tert-butyloxycarbonylamino-ethyl group or 2,2,2-trifluoro-1-benzyloxycarbonylamino-ethyl group [Chem. Ber., 100, pp. 3838-3849 (1967)] is preferably employed.

Among others preferably, X is an amino acid sequence consisting of optionally protected amino acid residues being selected from the group consisting of glutamyl, D-glutamyl, glycyl and lysyl.

In the formula (I), the amino acids in the optionally protected amino acid residues in the amino acid sequence shown by Y has the same meaning as that in the amino acid residue in the afore-mentioned amino acid sequence shown by X.

Especially preferable, Y is an amino acid sequence consisting of optionally protected amino acid residues being selected from the group consisting of glutamyl, glycyl, tyrosyl, lysyl and $\beta$-alanyl.

In the formula (I), the protecting group for Y has the same meaning as that in the afore-mentioned amino acid sequence shown by X.

In the formula (I), as the protecting groups shown by $R^3$, use is preferably made of protecting groups of the carboxy group described above.

In the formula (I), as the protecting groups shown by $R^4$, use is preferably made of protecting groups of the amino group described above.

In the formula (I), in the case where the amino acid residue is possibly an optically active isomer, it can take any of the L-, D- and DL-form.

In the following, description is made on the method of producing the above-mentioned compounds.

Protective groups and reagents frequently used are abbreviated as follows in the subsequent description.

Fmoc: 9-fluorenyl methyloxycarbonyl
Z : benzyloxy carbonyl
Boc : t-butoxy carbonyl
$^t$Bu : t-butyl
O$^t$Bu: t-butoxy
TFA : trifluoroacetic acid
TEA : triethylamine
DMF : N,N-dimethylformamide
DCC : N,N'-dicyclohexylcarbodiimide
BOP : benzotriazol-1-yloxy
tris(dimethylamino)phosphonium•hexafluorophosphate
DIC : N,N'-diisopropylcarbodiimide
HONB : N-hydroxy-5-norbornene-2,3-dicarboximide
HOBT : 1-hydroxybenzotriazole
DEPC : diethyl phosphorocyanidate
DCM : dichloromethane
THF : tetrahydrofuran
WSC : water-soluble carbodiimide hydrochloride
DMAP : 4-dimethylaminopyridine
MeOH : methanol
R- : R-configuration
S- : S-configuration

The compound, which is the basic skelton of the compounds in this specification is 2-amino-6,7-dihydroxy-4-thiaheptanoic acid.

```
                      OH
      3    4  5       |
      CH2-S-CH2-CH-CH2-OH
      |               6    7
H2N-CH-COOH
      2    1
```

In this specification, thiaheptanoyl is abbreviated as THT, thiaheptanoic acid is abbreviated THT-OH. And, n-hexadecanoyl is abbreviated as Pam, n-hexadecanoyloxy as PamO.

The compound represented by the formula (I) or a salt thereof, in which $R^3$ and $R^4$ is protecting group, can be produced by subjecting a material having a reactive carboxyl group corresponding to one of the several fragments separated at an optional position of the peptide linkage and a material having a reactive amino group corresponding to other fragment to condensation in order by a conventional means employed

in peptide synthesis.

As the conventional means of peptide synthesis, mention is made of, for example, anyone of liquid-phase synthetic method and solid-phase synthetic method. Such means of peptide synthesis as mentioned above include, for example, methods described in "Peptide Synthesis" written by M. Bondosky and M. Ondetti, Interscience, New York, 1966; "The Proteins" written by F.M. Finn and K. Hoffmann, Vol.2, edited by H. Nenrath and R.L. Hill, Academic Press Inc. New York, 1976; "Base & Experiment of Peptide Synthesis" written by IZUMIYA Nobuo, Maruzen Co. Ltd., 1985; "Seikagaku Jikken Koza 1" written by YAJIMA Haruaki, SAKAKIBARA Shunpei, et al. compiled by Japan Biochemistry Society, Tokyo Kagaku Dojin, 1977, "Zoku Seikagaku Jikken Koza 2" written by KIMURA Shun et al. compiled by Japan Biochemistry Society, Tokyo Kagaku Dojin, 1987; "Solid Phase Peptide Synthesis" written by J.M. Stewart and J.D. Young, Pizs chemical company, Illinois, 1984, or methods analogous to them. Practical examples of such methods as above includes, for example, azide method, chloride method, acid anhydride method, mixed acid anhydrides method, DCC method, active ester method, method using Woodword reagent K, carbonyl imidazole method, redoc method, DCC/HONB method, DIC/HONB method, WSC/HOBT method, method using BOP reagent, method using DEPC reagent or the like.

As the compound produced by activating the carboxyl group of the starting compound, mention is made of, for example, corresponding acid anhydrides, azides, active esters [e.g. esters with alcohol (e.g. pentachlorophenol, 2,4,5-trichlorophenol, 2,4-dinitrophenol, cyanomethyl alcohol, p-nitrophenol, N-hydroxy-5-norbornene-2,3-dicarboxyimde, N-hydroxysuccinimide, N-hydroxyphthalimide, N-hydroxybenzotriazole)]. As the starting compound whose amino group is activated, mention is made of, for example, the corresponding phosphoric acid amide.

The condensation reaction can be conducted in the presence of a solvent. The solvent can be selected from those known as being employable for peptide condensation reaction. Examples of the solvent include amides such as anhydrous or water-containing formamide, dimethylformamide, N-methyl pyrrolidone, etc., sulfoxides such as dimethyl sulfoxide, etc., aromatic amines such as pyridine, etc., halogenated hydrocarbons such as chloroform, dichloromethane, etc., ethers such as tetrahydrofuran, dioxane, etc., nitriles such as acetonitrile, etc., esters such as ethyl acetate, ethyl formate, etc., or a mixture of them in an appropriate ratio.

The reaction temperature can be adequately selected from the range known as being employable for peptide linkage forming reaction, specifically, for example, usually from about -20 °C to 40 °C.

The reaction time can be adequately selected from the range known as being required for peptide linkage formation reaction, specifically, for example, from about several minutes to about 48 hours.

As preferable practical examples of one of the stating fragments, in the case of producing the compound represented by the general formula (I) or a salt thereof, in which $R^3$ and $R^4$ is protecting group, mention is made of, for example, a compound represented by the general formula (II):

$H\text{-}X\text{-}OR^3$    (II)

wherein X and $R^3$ are of the same meanings as defined above or a salt thereof.

The compound (II) or salt thereof can be produced by, for example, the above-mentioned conventional means for peptide synthesis.

Preferable examples of the compound (II) include following compounds.

| Compound No. | Structural Formula |
|---|---|
| HP-1 | H-Gly-Gly-Gly-Glu(O$^t$Bu)-O$^t$Bu |
| HP-2 | H-Glu(O$^t$Bu)-Gly-D-Glu(O$^t$Bu)-O$^t$Bu |
| HP-3 | H-Gly-D-Glu(O$^t$Bu)-O$^t$Bu |
| HP-4 | H-Gly-Glu(O$^t$Bu)-Glu(O$^t$Bu)-O$^t$Bu |
| HP-5 | H-Glu(O$^t$Bu)-Gly-Glu(O$^t$Bu)-O$^t$Bu |
| HP-6 | H-Gly-Lys(Boc)-Gly-O$^t$Bu |

As the starting fragment for producing the compound (I) by combination with the above-mentioned compound (II), use is made of a compound represented by the formula (IV):

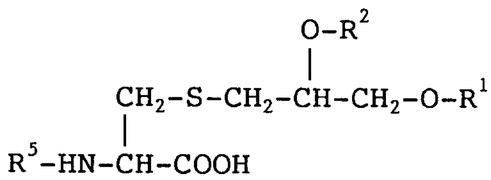

(IV)

wherein $R^5$ is an amino-protecting group; $R^1$ and $R^2$ are of the same meaning as defined above or a salt thereof.

The amino-protecting group represented by $R^5$ in the above-mentioned general formula (IV) has the same meaning as defined for the afore-described amino-protecting group.

The compound (IV) or a salt thereof, wherein $R^1$ and $R^2$ stand for one and the same group, can be produced be adequately applying a per se known method [e.g. International Journal of Peptide and Protein Research, 38, 1991, pp. 545-554; Chemical and Pharmaceutical Bulletin, 39, pp. 2590-2596]

In the case where $R^1$ and $R^2$ are different groups from each other, most common production route is described below.

First, monoacyl compound of a glycerin derivative, for example, glycidol is prepared. On the other hand, the disulfide linkage of cystine in which amino group and carboxyl group are protected is opened reductively to give a cystine derivative.

By subjecting this cysteine derivative to addition reaction to the above-mentioned monoacyl glycerine derivative, 2-amino-6-hydroxy-7-acyloxy-4-thiaheptanoic acid derivative, while use of S-(-)-glycidol gives a (6S)-2-amino-6-hydroxy-7-acyloxy-4-thiaheptanoic acid in which the amino group and carboxyl group are protected.

In this reaction, use of R-(+)-glycidol gives a (6R)-2-amino-6-hydroxy-7-acyloxy-4-thiaheptanoic acid derivative, while use of S-(-)-glycidol gives a (6S)-2-amino-6-hydroxy-7-acyloxy-4-thiaheptanoic acid derivative.

By conventional acylation of the hydroxyl group at 6-position of 2-amino-6-hydroxy-7-acyloxy-4-thiaheptanoic acid obtained by the above-mentioned reaction, a 2-amino-6,7-bis(acyloxy)-4-thiaheptanoic acid derivative having different O-acyl group can be obtained.

By directly using, for example, glycidol instead of an acyl glycerin derivative, 2-amino-6,7-dihydroxy-4-thiaheptanoic acid derivative is obtained, and, acylation of this product by a conventional method conveniently gives 2-amino-6,7-bis(acyloxy)-4-thiaheptanoic acid derivative having the same O-acyl group.

By removing, in the later-mentioned manner, the protecting group of the carboxyl group of the 2-amino-6,7-bis(acyloxy)-4-thiaheptanoic acid derivatives thus obtained, 2-amino-6,7-bis(acyloxy)-4-thiaheptanoic acid having protected amino group can be prepared.

Preferable examples of the compound (IV) include 2-amino-6,7-bis(acyloxy)-4-thiaheptanoic acid having protected amino group. Further example is shown below:

| Compound No. | Reference Example No. | Structural formula |
|---|---|---|
| FmocGC-1 | 8 | (2R,6R)-2-Fmoc-amino-6,7-bis(PamO)-4-THT-OH |

The compound (II) and compound (IV) obtained thus above are subjected to condensation in the following manner for peptide synthesis, and then, if necessary, the amino-protecting group shown by $R^5$ is removed by the manner described above to give the compound (III):

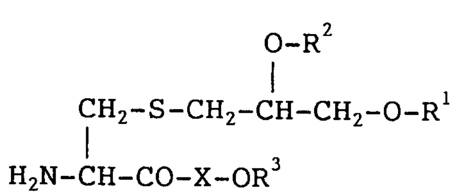

(III)

wherein $R^1$ and $R^2$ are of the same meaning as defined above or a salt thereof.

Concrete examples are shown below:

| Compound No. | Reference Example No. | Structural formula |
|---|---|---|
| HGCP-1 | 9 | (2R,6R)-2-Amino-6,7-bis(PamO)-4-THT-Gly-Gly-Gly-Glu-$O^tBu$ |
| HGCP-2 | 10 | (2R,6R)-2-Amino-6,7-bis(PamO)-4-THT-Glu($O^tBu$)-Gly-D-Glu($O^tBu$)-$O^tBu$ |
| HGCP-3 | 11 | (2R,6R)-2-Amino-6,7-bis(PamO)-4-THT-Gly-D-Glu($O^tBu$)-$O^tBu$ |
| HGCP-4 | 12 | (2R,6R)-2-Amino-6,7-bis(PamO)-4-THT-Gly-Glu($O^tBu$)-Glu($O^tBu$)-$O^tBu$ |
| HGCP-5 | 13 | (2R,6R)-2-Amino-6,7-bis(PamO)-4-THT-Glu($O^tBu$)-Gly-Glu($O^tBu$)-$O^tBu$ |
| HGCP-6 | 14 | (2R,6R)-2-Amino-6,7-bis(PamO)-4-THT-Gly-Lys(Boc)-Gly-$O^tBu$ |

The compound represented by the general formula (I) or a salt thereof, in which $R^3$ and $R^4$ is protecting group, can be produced by subjecting the compound (III) or a salt thereof and the compound represented by the general formula (V) to condensation by the conventional means for peptide synthesis.

$R^4$-Y-OH (V)

Wherein $R^4$ and Y are of the same meaning as defined above.

Where Y represents an amino acid sequence consisting of 2 to 5 amino acid residues which may be substituted, the desired compound or salt can be obtained by subjecting said compound (III) or a salt thereof to serial condensation with peptide fragments or amino acids which are to constitute compound (V).

The resulting compound (I) or salt wherein $R^3$ and $R^4$ each represents a protective group can then be deprotected to give a compound of general formula (I) or a salt thereof wherein $R^3$ and $R^4$ each represents hydrogen.

The above deprotection reaction can be conducted by a per se known method, for example, a method conventionally employed in the field of peptide chemistry. [cf. Synthetic Chemistry Series, Peptide Synthesis, Authors: ISUMIYA Nobuo, ONO Motonari, KATO Tetsuo & AOYAGI Haruhiko; Published by Maruzen Co. Ltd. 1975 in Japan].

Deprotection reaction on the amino group protected with a urethane-type protecting group is conducted by bringing the amino group into contact with an acid in the absence of solvent or in a solvent which gives no adverse influence on the reaction. As the solvent, use is made of halogenated hydrocarbons (e.g. dichloromethane, chloroform, 1,2-dichloroethane, etc.), alcohols (e.g. methanol, ethanol, etc.), water and a mixture of them in appropriate ratio. As the acid, use is made of, for example, haloacetic acid (e.g. trifluoroacetic acid, etc.), hydrohalic acid (e.g. hydrochloric acid, hydrobromic acid, etc.), among others.

It is advantageous that N-benzyloxycarbonyl group and N-4-methoxybenzyloxycarbonyl group are removed by catalytic hydrogenation by using, for example, palladium catalyst (e.g. palladium carbon, palladium barium sulfate, palladium black, etc.) or rhodium catalyst. In this case, a known solvent, for example, cyclic ether (e.g. tetrahydrofuran etc.), alcohols (e.g. methanol, ethanol, etc.) etc., or, depending on cases, a mixture of cyclic ether and other inert solvents [e.g. lower aliphatic acid amides (e.g. dimethylformamide, etc.) etc.] is used.

N-9-fluorenylmethyloxycarbonyl group is removed advantagenously by using an organic amine, for example, diethylamine, piperidine, morpholine, 4-dimethylaminopyridine or dicyclohexylamine or the like. The reaction is conducted in a solvent which gives no adverse affect on the reaction. As the solvent, use is made of, for example, amides (e.g. dimethylformamide, acetamide, etc.), alcohols (e.g. methanol, ethanol, etc.), halogenated hydrocarbons (e.g. dichloromethane, chloroform, 1,2-dichloroethane, etc.), etc., or a mixture of them in an appropriate ratio.

It is advantageous that, N-2,2,2-trichloroethyloxycarbonyl group is removed by using a metal (e.g. zinc etc.) together with an organic carboxylic acid (e.g. acetic acid, propionic acid, etc.). The reaction is conducted in a solvent which gives no adverse influence on the reaction. As the solvent, use is made of the above-mentioned carboxylic acid, alcohols (e.g. methanol, ethanol, etc.), water or a mixture of them in an appropriate ratio.

Deprotection reaction (deacylation reaction) of acylated hydroxy group is conducted by bringing it into contact with an acid in a solvent which gives no adverse influence. As the solvent, use is made of halogenated hydrocarbons (e.g. dichloromethane, chloroform, 1,2-dichloroethane, etc.), alcohols (e.g. methanol, ethanol, etc.), water and a mixture of them in an appropriate ratio. As the acid, use is made of, for example, haloacetic acid (e.g. trifluoroacetic acid, etc.), hydrohalogenic acid (e.g. hydrochloric acid, hydrobromic acid, etc.), etc..

Elimination of O-benzyl group is performed advantageously by catalytic hydrogenation with, for example, a palladium catalyst (e.g. palladium carbon, palladium/barium sulfate and palladium black, etc.) or

a rhodium catalyst, using, in this case, a known solvent, for example, cyclic ether (e.g. tetrahydrofuran etc.), alcohols (e.g. methanol, ethanol, etc.) etc., or, depending on cases, a mixture of cyclic ether and other inert solvents [e.g. lower aliphatic acid amides (e.g. dimethylformamide etc.) etc.] is used.

Elimination of O-tetrahydropyranyl group or O-tert-butyl group can be performed, like in the above-mentioned deacylation, by hydrolysis with an acid.

Elimination of a carboxy-protecting group can be performed, like in the above-mentioned case, by hydrolysis with an acid. And, benzyl ester, for example, can be deprotecting by catalytic hydrogenation like in the case of the above-mentioned deprotection of the O-benzyl group elimination. The above-mentioned 2-(trimethylsilyl)ethyl group can be eliminated by the action of, for example, a salt of hydrofluoric acid, for example, especially a salt of a quaternary nitrogen base with hydrofluoric acid (e.g. tetraethyl ammonium fluoride) in a suitable solvent under neutral conditions.

The compound (I) or salt thereof thus produced can be recovered, after completion of the reaction, by means for separating peptide, for example, extraction, partition, reprecipitation, crystallization, recrystallization, various chromatographic processes, high performance liquid chromatography and so on.

A salt of the compound (I) of this invention with a base, especially with a pharmaceutically acceptable base, can be obtained by a per se known method. Examples of the base include on alkali metal such as sodium, potassium, etc., an alkaline earth metal such as calcium, magnesium, etc., an organic base such as tertiary amines e.g. triethylamine, piridine, etc. and so on. A salt of the compound (I) of this invention with an acid, especially with a pharmaceutically acceptable acid, can be obtained by a per se known method. Examples of the acid include, an inorganic acid (e.g. hydrochloric acid, sulfuric acid, phosphoric acid, etc.), an organic acid (e.g. acetic acid, propionic acid, citric acid, tartaric acid, malic acid, oxalic acid, etc.) and so on.

And, as salts of the compounds (II) to (V), use is made of those substantially the same as salts of the compound (I).

The compound (I) or a salt thereof have activities of remarkably improving the state of hematopoiesis-insufficiency, which can be used as therapeutic or prophylactic agents of leukocytopenia caused by radiotherapy or chemotherapy of cancers in mammals (e.g. man, macaque, horse, cow, pig, dog, etc.), as hematopoietic enhancing agents in the case of bone marrow transplantation, as immuno-enhancing agents having leukocyte-increasing action, and, further, as a therapeutic agent of thrombocytopenia.

The compound (I) or a salt thereof are of low in toxicity and can be used safely.

In the case of administering the compound (I) or a salt thereof to, for example, man, it can be safely administered orally or non-orally alone or as a pharmaceutical composition by mixing with a suitable pharmaceutically acceptable carrier, excipient or diluent.

Example of the above-mentioned pharmaceutical composition include orally administrable compositions (e.g. powder, granules, capsules, tablets), injections, drip injections, topica (e.g. transnasal agent, transdermal agents, etc.), suppositories (e.g. rectal suppositories, vaginal suppositories).

These pharmaceutical composition can be prepared by per se known methods generally employed in the processes of pharmaceutical preparation.

For example, the compound (I) or salt thereof of this invention can be prepared into aqueous injections together with a dispersant [e.g. Tween 80 (manufactured by Atlas Powder Co., U.S.A.), HCO 60 (manufactured by Nikko Chemicals), polyethylene glycol, carboxymethyl cellulose, sodium alginate or the like], a preservative (e.g. methylparaben, propylparaben, benzyl alcohol or the like) and an isotonicating agent (e.g. sodium chloride, mannitol, sorbitol, glucose or the like), among others, or into oleaginous injections by dissolving, suspending or emulsifying in a vegetable oil such as olive oil, sesame oil, cotton seed oil, corn oil or the like, propylene glycol, among others.

For preparing the compound (I) or a salt thereof of this invention into compositions for oral administration, it is subjected to compression molding, in accordance with a per se known method, together with an excipient (e.g. lactose, sucrose, starch or the like), a disintegrator (e.g. starch, calcium carbonate or the like), a binding agent (e.g. starch, gum arabica, carboxymethyl cellulose, polyvinyl pyrrolidone, hydroxypropyl cellulose or the like) or a lubricant (e.g. talc, magnesium stearate, polyethylene glycol 6000), etc., followed by, upon necessity, masking the taste or coating by a per se known process for the purpose of enteric coating or of making the compositions to be of sustained-release. Examples of the coating agent include hydroxypropyl methyl cellulose, ethyl cellulose, hydroxymethyl cellulose, hydroxypropyl cellulose, polyoxyethylene glycol, Tween 80, Brulonick F68, cellulose acetate phthalate, hydroxypropyl methyl cellulose phthalate, hydroxymethyl cellulose acetate succinate, Eudragit (manufactured by Rohm, Germany, methacrylic acid-acrylic acid copolymer) and a pigment such as titanium oxide, red iron oxide etc. Between such an enteric coating layer and the core layer there may be provided an intermediate layer.

For preparing the compound (I) or a salt thereof into, for example, solid, semi-solid or liquid compositions for topical use, a per se known method can be resorted to. For preparing solid compositions, for examples, the compound (I) or a salt thereof is prepared into powdery compositions singly or in admixutre with an excipient (e.g. glycol, mannitol, starch, microcrystalline cellulose or the like), a thickening agent (e.g. natural rubbers, cellulose derivatives, acrylic acid polymers or the like). As the above mentioned liquid composition, almost like in the case of injections, mention is made of oleaginous or aqueous suspensions. As the semi-solid composition, aqueous or oleaginous gel compositions or ointments are preferably counted. These compositions may be supplemented with a pH regulator (e.g. carbonic acid, phosphoric acid, citric acid, hydrochloric acid, sodium hydroxide, etc.), a preservative (e.g. p-hydroxybenzoic esters, chlorobutanol, benzalkonium chloride, etc.), among others.

In the case of preparing suppositories for examples, the compound (I) or a salt thereof of the present invention can be prepared into oleaginous or water-soluble solid, semi-solid or liquid suppositories. As the oleaginous base for the above-mentioned compositions, any one can be employed, as exemplified by the glycerides of higher fatty acid [e.g. cacao butter, Wittepsols (manufactured by Dynamite Nobel, Inc.), etc.], middle class fatty acid [e.g. Migliols (manufactured by Dynamite Nobel, Inc.), etc.] or vegetable oil (e.g. sesame oil, soybean oil, cotton seed oil, etc.), among others. And, examples of the water-soluble base include polyethylene glycols and propylene glycols, and, examples of the water-soluble gel base include natural rubbers, cellulose derivatives, vinyl polymers, acrylic acid polymers, etc.

While the dosage of the compound (I) or a salt thereof when administered to man as injections varies with diseases, administration routes, ages of individual patients and states of disease, it ranges from about 0.05 $\mu$g to 50 mg in terms of the effective component, preferably from about 0.5 $\mu$g to 10 mg, more preferably from about 2.5 $\mu$g to 0.25 mg in the case of a common adult patient (50 kg body weight).

[Examples]

By the following reference examples, working examples, experimental examples and formulation examples, the present invention will be described in further detail, but they are not intended to limit the invention in any manner. The numerals showing the mixing ratio in mixed solvents mean the volume ratio of each solvent.

$^1$H-NMR spectrum is determined by a Varian Gemini 200 (200 MHZ) type spectrometer using tetramethylsilane as the internal standard, expressing all the $\delta$ values as ppm.

Symbols used in the specification are of the following meaning.

S: singlet, d: doublet, t: triplet, q: quartet, dd: double doublet, dt: double triplet, m: multiplet, br: broad.

Reference Example 1

Production of Z-Gly-Gly-Gly-OH

H-Gly-Gly-Gly-OH (10.0 g, 52.9 mmol, Peptide Laboratories, Japan) was dissolved in 4N aqueous solution of sodium hydroxide (13.3 ml). To the solution were added under ice-cooling, 4N aqueous solution of sodium hydroxide (15.9 ml) and benzyloxy carbonyl chloride (9.31 ml), then the mixture was stirred at 20 °C for over night. The reaction mixture was washed with ether, then the aqueous layer was adjusted to pH 3.0 with 5N HCl under ice-cooling, which was left standing at cold room for over night. Resulting crystals were collected by filtration, which were washed with cold water, followed by drying. The product was used in following reference example without purification.

Reference Example 2

Production of H-Gly-Gly-Gly-Glu(O$^t$Bu)-O$^t$Bu (HP-1)

a) Z-Gly-Gly-Gly-OH (0.84 g, 2.6 mmol) was dissolved in DMF (15 ml). To the solution were added, H-Glu(OtBu)-OtBu (0.68 g, 2.6 mmol), HOBT (386 mg, 2.9 mmol) and WSC (548 mg, 2.9 mmol), then the mixture was stirred at room temperature for 18 hours. The reaction mixture was concentrated, which was suspended in 0.2 M citric acid-water (100 ml), followed by extraction with ethyl acetate (100 ml) twice. The ethyl acetate layers were combined and washed with a 10% (w/v) aqueous solution of ammonium chloride, 5% (w/v) aqueous solution of sodium hydrogencarbonate, water and a saturated aqueous saline solution, successively, which was dried over anhydrous sodium sulfate, followed by concentration. To the concentrate was added ether-hexane. Resulting precipitate were collected by filtration to afford Z-Gly-Gly-Gly-Glu(O$^t$Bu)-O$^t$Bu (ZP-1) as a white powder product (0.81 g, yield 55%).
$[\alpha]_D^{23}$ + 9.6° (c = 0.56, in chloroform)

| Elemental analysis for $C_{27}H_{40}N_4O_9$: | | | |
|---|---|---|---|
| Calcd.<br>Found | C, 57.43;<br>C, 57.60; | H, 7.14;<br>H, 7.12; | N, 9.92<br>N, 9.88 |

b) This powdery product (735 mg) was dissolved in methanol (25 ml), to which was added 10% (w/w; the same applies hereinafter) palladium-carbon (73 mg). The mixture was stirred in a hydrogen stream at ordinary temperature under normal pressure for 2 hours. The catalyst was removed, and the solvent was distilled off to leave HP-1 as powder product (543 mg).
$[\alpha]_D^{23}$ + 13.1° (c = 0.51, in chloroform)

| Elemental analysis for $C_{19}H_{34}N_4O_7 \cdot 0.5H_2O$: | | | |
|---|---|---|---|
| Calcd.<br>Found | C, 51.92;<br>C, 52.09; | H, 8.03;<br>H, 7.89; | N, 12.75<br>N, 12.56 |

Reference Example 3

Production of H-Glu(O$^t$Bu)-Gly-D-Glu(O$^t$Bu) (HP-2)

In substantially the same manner as in Reference Example 2, H-Glu(O$^t$Bu)-Gly-D-Glu(O$^t$Bu)-O$^t$Bu (HP-2) was produced.

| Materials | | Reaction Conditions | | Product |
|---|---|---|---|---|
| a) | 1) Fmoc-Glu(O$^t$Bu)-OH (2.78 g) | WSC<br>HOBT<br>DCM | 1.24 g<br>876 mg<br>85 ml | Fmocp-2 (3.73 g) |
| | 2) H-Gly-D-Glu(O$^t$Bu)-O$^t$Bu (1.86 g) | 20°C | 16 h | |

$[\alpha]_D^{23}$ - 12° (c = 0.54, in chloroform)

| Elemental analysis for $C_{39}H_{53}N_3O_{10}$: | | | |
|---|---|---|---|
| Calcd.<br>Found | C, 64.71;<br>C, 64.55; | H, 7.38;<br>H, 7.43; | N, 5.80<br>N, 6.09 |

b) This powdery product (2.10 g) was dissolved in DCM (63 ml), to which was added piperidine (7.0 ml), and the mixture was stirred at 20°C for 1 hour. To the reaction mixture was added ethyl acetate (300 ml), which was subjected to extraction with 1N HCl (70 ml) and 0.03 N HCl (100 ml x 2). The aqueous layer was adjusted to pH 6.0, which was washed with a mixture of hexane: ether (=1:1) (100 ml x 5). Then, the pH was adjusted to 8.6, followed by extraction with ethyl acetate (150 ml x 2). The extract was dried over anhydrous sodium sulfate, followed by concentration to afford HP-2 as a colorless oily product (1.31 g).
$[\alpha]_D^{23}$ - 17° (c = 0.47, in chloroform)

| Elemental analysis for $C_{24}H_{43}N_3O_8$: | | | |
|---|---|---|---|
| Calcd.<br>Found | C, 57.47;<br>C, 57.40; | H, 8.64;<br>H, 8.73; | N, 8.38<br>N, 8.60 |

Reference Example 4

Production of H-Gly-D-Glu(O$^t$Bu)-O$^t$Bu (HP-3)

In substantially the same manner as in Reference Example 2, H-Gly-D-Glu(O$^t$Bu)-O$^t$Bu (HP-3) was produced.

| Materials | | Reaction Conditions | | Products |
|---|---|---|---|---|
| a) | 1) Z-Gly-OH (620 mg) | WSC<br>HOBT<br>DCM | 569 mg<br>401 mg<br>23 ml | ZP-3 (1.23 g) |
| | 2) H-D-Glu(O$^t$Bu)-O$^t$Bu (700 mg) | 20 °C | 16 h | |
| b) | ZP-3 (1.23 g) | 10% Pd-C<br>MeOH (methanol; the same applies hereinafter)<br>20 °C | 120 mg<br>40 ml<br><br>1.5 h | HP-3 (830 mg) |

Compound ZP-3: $[\alpha]_D^{20}$ - 10.3° (c = 0.52, in chloroform)

| Elemental analysis for $C_{23}H_{34}N_2O_7 \cdot 0.25H_2O$: | | | |
|---|---|---|---|
| Calcd.<br>Found | C, 60.71;<br>C, 60.79; | H, 7.64;<br>H, 7.68; | N, 6.16<br>N, 6.28 |

Compound HP-3: $[\alpha]_D^{20}$ - 17.9° (c = 0.52, in chloroform)

| Elemental analysis for $C_{15}H_{28}N_2O_5$: | | | |
|---|---|---|---|
| Calcd.<br>Found | C, 56.94;<br>C, 56.81; | H, 8.92;<br>H, 8.95; | N, 8.85<br>N, 9.04 |

Reference Example 5

Production of H-Gly-Glu(O$^t$Bu)-Glu(O$^t$Bu)-O$^t$Bu (HP-4)

In substantially the same manner as in Reference Example 2, H-Gly-Glu(O$^t$Bu)-Glu(O$^t$Bu)-O$^t$Bu (HP-4) was produced.

| Materials | | Reaction Conditions | | Products |
|---|---|---|---|---|
| a) | 1) Z-Gly-OH (347 mg) | WSC<br>HOBT<br>DCM | 318 mg<br>224 mg<br>22 ml | ZP-4 (930mg) |
| | 2) H-Glu(O$^t$Bu)-Glu(O$^t$Bu)-O$^t$Bu (670 mg) | 20 °C | 16 hr | |
| b) | ZP-4 (870 mg) | 10% Pd-C<br>MeOH<br>20 °C | 90 mg<br>30 ml<br>1.5 h | HP-4 (650 mg) |

Compound ZP-4: m.p. 92.4-92.7 °C
$[\alpha]_D^{24}$ - 3.5° (c = 0.51, in chloroform)

| Elemental analysis for $C_{32}H_{49}N_3O_{10} \cdot 0.25H_2O$: | | | |
|---|---|---|---|
| Calcd. | C, 60.03; | H, 7.79; | N, 6.56 |
| Found | C, 60.18; | H, 7.81; | N, 6.26 |

Compound HP-4: $[\alpha]_D^{24}$ - 6.8° (c=0.54, in chloroform)

| Elemental analysis for $C_{24}H_{43}N_3O_8 \cdot 0.25H_2O$: | | | |
|---|---|---|---|
| Calcd. | C, 56.95; | H, 8.66; | N, 8.30 |
| Found | C, 56.92; | H, 8.74; | N, 8.06 |

Reference Example 6

Production of H-Glu(O$^t$Bu)-Gly-Glu(O$^t$Bu)-O$^t$Bu (HP-5)

In substantially the same manner as in Reference Example 2, H-Glu(O$^t$Bu)-Gly-Glu(O$^t$Bu)-O$^t$Bu (HP-5) was produced.

| Materials | | Reaction Conditions | | Products |
|---|---|---|---|---|
| a) | 1) Z-Glu(O$^t$Bu)-OH (785 mg) | WSC<br>HOBT<br>DCM | 447 mg<br>315 mg<br>22 ml | ZP-5 (1.20 g) |
| | 2) H-Gly-Glu(O$^t$Bu)-O$^t$Bu (670 mg) | 20°C | 16 hr | |
| b) | ZP-5 (1.10 g) | 10% Pd-C<br>MeOH<br>20°C | 110 mg<br>40 ml<br>2 h | HP-5 (840 mg) |

Compound ZP-5: $[\alpha]_D^{24}$ + 4.2° (c=0.53, in chloroform)

| Elemental analysis for $C_{32}H_{49}N_3O_{10}$: | | | |
|---|---|---|---|
| Calcd. | C, 60.46; | H, 7.77; | N, 6.61 |
| Found | C, 60.48; | H, 7.78; | N, 6.88 |

Compound HP-5: $[\alpha]_D^{24}$ + 7.0° (c=0.52, in chloroform)

| Elemental analysis for $C_{24}H_{43}N_3O_8 \cdot 0.25H_2O$: | | | |
|---|---|---|---|
| Calcd. | C, 56.95; | H, 8.66; | N, 8.30 |
| Found | C, 56.94; | H, 8.60; | N, 8.04 |

Reference Example 7

Production of H-Gly-Lys(Boc)-Gly-O$^t$Bu (HP-6)

In substantially the same manner as Reference Example 2, H-Gly-Lys(Boc)-Gly-O$^t$Bu (HP-6) was synthesized.

| Materials | | Reaction Conditions | | Products |
|---|---|---|---|---|
| a) | 1) Z-Gly-OH (813 mg) | WSC<br>HOBT<br>DMF | 745 mg<br>525 mg<br>20 ml | ZP-6 (1.90 g) |
| | 2) H-Lys(Boc)-Gly-O$^t$Bu (1.27 g) | 20 ° C | 13 h | |
| b) | ZP-6 (1.54 g) | 10% Pd/C<br>MeOH<br>20 ° C | 154 mg<br>40 ml<br>5 h | HP-6 (1.15 g) |

Compound ZP-6: $[\alpha]_D^{24}$ - 13.4° (c=0.58, in chloroform)

| Elemental analysis for $C_{27}H_{42}N_4O_8 \cdot 0.5H_2O$: | | | |
|---|---|---|---|
| Calcd.<br>Found | C, 57.95;<br>C, 57.73; | H, 7.74;<br>H, 7.44; | N, 10.01<br>N, 10.03 |

Compound HP-6: $[\alpha]_D^{24}$ - 23.8° (c=0.58, in chloroform)

| Elemental analysis for $C_{19}H_{36}N_4O_6 \cdot H_2O$: | | | |
|---|---|---|---|
| Calcd.<br>Found | C, 52.52;<br>C, 52.72; | H, 8.81;<br>H, 8.81; | N, 12.89<br>N, 12.86 |

Reference Example 8

Production of (2R,6R)-2-Fmoc-amino-6,7-bis(PamO)-4-THT-OH (FmocGC-1)

a) (Fmoc-(R)-Cys-O$^t$Bu)$_2$ (10.0 g, 12.5 mmol) prepared by the method described in a literature (J.W. Metzger et al., Int. J. Peptide Protein Res. 38, 545, 1991) was dissolved in DCM (80 ml). To the solution were added under ice-cooling, powdery zinc (3.27 g, 50.0 mmol) and a mixture of MeOH-36% (w/v) HCl-conc. $H_2SO_4$ (100:7:1) (hereinafter simply referred to as "acid mixture solution") (40 ml). The resultant mixture was stirred at 20° C for 20 minutes. To the reaction mixture was added (R)-(+)-glycidol (8.29 ml, 125 mmol), which was stirred at 40° C for 2.5 hours. The reaction mixture was concentrated until its volume is reduced to 50 ml, then insolubles were filtered off. To the filtrate was added a saturated aqueous saline solution (200 ml), and the mixture was subjected to extraction with DCM (2 x 300 ml). DCM layers were combined, dried over anhydrous sodium sulfate, and then concentrated. The concentrate was subjected to a silica-gel column chromatography, eluting with ethyl acetate-hexane (1:1, 3:1), successively. Fractions containing the object compound were combined and concentrated to give (2R,6R)-2-Fmoc-amino-6,7-dihydroxy-4-THT-O$^t$Bu (FmocGC-1a) as a white powdery product (10.9 g, yield 92%).
$[\alpha]_D^{21}$ - 8.8° (c=0.65, in chloroform)

| Elemental analysis for $C_{25}H_{31}NO_6S \cdot 0.5H_2O$: | | | | |
|---|---|---|---|---|
| Calcd.<br>Found | C, 62.22;<br>C, 62.14; | H, 6.68;<br>H, 6.66; | N, 2.90;<br>N, 2.81; | S, 6.64<br>S, 6.54 |

b) The compound, FmocGC-1a (3.90 g, 8.23 mmol) was dissolved in THF (70 ml), to which were added palmitic acid (6.33 g, 24.7 mmol), DIC (3.87 ml, 24.7 mmol) and 4-dimethylaminopyridine (DMAP, 402 mg, 3.29 mmol). The mixture was stirred at 20° C for 13 hours. The mixture was concentrated, and the residue was suspended in 10% (w/v) aqueous solution of citric acid (200 ml). The suspension was subjected to extraction with ethyl acetate (400 ml). The ethyl acetate layer was washed with water, and then concentrated. The concentrate was subjected to a silica-gel column chromatography, eluting with

ethyl acetate-hexane mixtures (1:20, 1:10), successively. Fractions containing the object compound were combined and concentrated. The concentrate was crystallized from hexane to give (2R,6R)-2-Fmoc-amino-6,7-bis(PamO)-4-THT-$O^tBu$ (FmocGC-1b) as colorless crystals (4.76 g, yield 61%).
m.p. 58.0 °C

| Elemental analysis for $C_{57}H_{91}NO_8S$: | | | | |
|---|---|---|---|---|
| Calcd. | C, 72.03; | H, 9.65; | N, 1.47; | S, 3.37 |
| Found | C, 71.94; | H, 9.58; | N, 1.43; | S, 3.36 |

c) The compound FmocGC-1b (2.50 g, 2.63 mmol) was dissolved in TFA (50 ml), and the solution was left standing at 20 °C for 1.5 hours. The reaction mixture was concentrated, and the concentrate was dissolved in ethyl acetate (500 ml). This solution was washed with water, dried over anhydrous sodium sulfate, followed by concentrated. Resulting crystals were recrystallized from ethyl acetate to afford FmocGC-1 as colorless crystals (2.20 g, yield 94%).
m.p. 90.0 °C
$[\alpha]_D^{20}$ + 12.9 ° (c = 0.73, in chloroform)

| Elemental analysis for $C_{53}H_{83}NO_8S$: | | | | |
|---|---|---|---|---|
| Calcd. | C, 71.18; | H, 9.35; | N, 1.57; | S, 3.59 |
| Found | C, 71.23; | H, 9.12; | N, 1.54; | S, 3.47 |

Reference Example 9

Production of (2R,6R)-2-amino-6,7-bis(PamO)-4-THT-Gly-Gly-Gly-Glu-OH (HGCP-1)

a) The compound FmocGC-1 (1.31 g) synthesized in reference Example 8 was dissolved in DMF (19 ml), to which were added, under ice-cooling, HONB (288 mg), DIC (250 µl) and H-Gly-Gly-Gly-Glu-($O^tBu$)-$O^tBu$(HP-1) (630 mg) obtained in Reference Example 2. The reaction mixture was stirred at 20 °C for 15 hours. This reaction mixture was concentrated, which was then dissolved in ethyl acetate. The solution was washed with 0.2M aqueous solution of citric acid, 10% (w/v) aqueous solution of ammonium chloride, 5% (w/v) aqueous solution of sodium hydrogencarbonate, water and saturated aqueous saline solution successively. The ethyl acetate layer was dried over anhydrous sodium sulfate, followed by concentration. To the concentrate was added acetonitrile. Resulting precipitate were collected by filtration to afford (2R,6R)-2-Fmoc-amino-6,7-bis(PamO)-4-THT-Gly-Gly-Gly-Glu($O^tBu$)-$O^tBu$(FmocGCP-1) as white powder product (1.64 g, yield 86%).
$[\alpha]_D^{23}$ - 6.4 ° (c = 0.50, in chloroform)

| Elemental analysis for $C_{72}H_{115}N_5O_{14}S$: | | | | |
|---|---|---|---|---|
| Calcd. | C, 66.18; | H, 8.87; | N, 5.36; | S, 2.45 |
| Found | C, 66.03; | H, 8.87; | N, 5.31; | S, 2.22 |

b) This powdery product FmocGCP-1 (1.18 g) was dissolved in DCM (12 ml), to which was added piperidine (1.2 ml). The mixture was stirred at 20 °C for 1.3 hours. This reaction mixture was then concentrated. The concentrate was subjected to a silica-gel column chromatography, eluting with chloroform-methanol mixtures (49:1, 19:1, 14:1) successively. Fractions containing the object compound were combined and concentrated to leave (2R,6R)-2-amino-6,7-bis(PamO)-4-THT-Gly-Gly-Gly-Glu($O^tBu$)-$O^tBu$ (HGCP-1) as white powdery product (0.92 g, yield 94%).
$[\alpha]_D^{23}$ - 6.7 ° (c = 0.63, in chloroform)

| Elemental analysis for $C_{57}H_{105}N_5O_{12}S \cdot H_2O$: | | | | |
|---|---|---|---|---|
| Calcd. | C, 62.09; | H, 9.78; | N, 6.35; | S, 2.91 |
| Found | C, 62.12; | H, 9.59; | N, 6.36; | S, 2.87 |

Reference Example 10

Production of (2R,6R)-2-Fmoc-amino-6,7-bis(PamO)-4-THT-Glu($O^tBu$)-Gly-D-Glu($O^tBu$)-$O^tBu$ (FmocGCP-2) and (2R,6R)-2-amino-6,7-bis(PamO)-4-THT-Glu($O^tBu$)-Gly-D-Glu($O^tBu$)-$O^tBu$ (HGCP-2)

In substantially the same manner as in Reference Example 9, (2R,6R)-2-Fmoc-amino-6,7-bis(PamO)-4-THT-Glu($O^tBu$)-Gly-D-Glu($O^tBu$)-$O^tBu$ (FmocGCP-2) and (2R,6R)-2-amino-6,7-bis(PamO)-4-THT-Glu($O^tBu$)-Gly-D-Glu($O^tBu$)-$O^tBu$ (HGCP-2) were produced.

| Materials (g) | Reaction Conditions | | Products (g) |
|---|---|---|---|
| a) FmocGC-1 (2.04) | HP-2<br>HONB<br>DIC<br>DMF<br>20°C | 1.31 g<br>453 mg<br>0.40 ml<br>20 ml<br>16 h | FmocGCP-2 (2.72) |
| b) FmocGCP-2 (2.00) | Piperidine<br>DCM<br>20°C<br>Silica gel (ethyl acetate-methanol) 10:0, 9:1 | 2.0 ml<br>18 ml<br>1 h | HGCP-2 (1.75) |

Compound FmocGCP-2: $[\alpha]_D^{23}$ - 7.0° (c = 0.56, in chloroform)

| Elemental analysis for $C_{77}H_{124}N_4O_{15}S$: | | | | |
|---|---|---|---|---|
| Calcd. | C, 67.12; | H, 9.07; | N, 4.07; | S, 2.33 |
| Found | C, 67.01; | H, 9.19; | N, 4.01; | S, 2.31 |

Compound HGCP-2: $[\alpha]_D^{23}$ - 18° (c = 0.53, in chloroform)

| Elemental analysis for $C_{62}H_{114}N_4O_{13}S$: | | | | |
|---|---|---|---|---|
| Calcd. | C, 64.44; | H, 9.94; | N, 4.85; | S, 2.77 |
| Found | C, 64.23; | H, 9.95; | N, 4.94; | S, 2.64 |

Reference Example 11

Production of (2R,6R)-2-Fmoc-amino-6,7-bis(PamO)-4-THT-Gly-D-Glu($O^tBu$)-$O^tBu$(FmocGCP-3) and (2R,6R)-2-amino-6,7-bis(PamO)-4-THT-Gly-D-Glu($O^tBu$)-$O^tBu$ (HGCP-3)

In substantially the same manner as in Reference Example 9, (2R,6R)-2-Fmoc-amino-6,7-bis(PamO)-4-THT-Gly-D-Glu($O^tBu$)-$O^tBu$ (FmocGCP-3) and (2R,6R)-2-amino-6,7-bis(PamO)-4-THT-Gly-D-Glu($O^tBu$)-$O^tBu$ (HGCP-3) were produced.

| Materials (g) | | Reaction Conditions | | Products (g) |
|---|---|---|---|---|
| a) | FmocGC-1 (2.12) | HP-3<br>HONB<br>DIC<br>DMF<br>20 °C | 830 mg<br>471 mg<br>412 µl<br>21 ml<br>16 h | FmocGCP-3 (2.18) |
| b) | FmocGCP-2 (2.00) | Piperidine<br>DCM<br>20 °C<br>Silica gel (chloroform-methanol) 97:3 | 2.0 ml<br>18 ml<br>1 h | HGCP-3 (1.72) |

Compound FmocGCP-3: $[\alpha]_D^{24}$ - 12.5° (c = 0.55, in chloroform)

| Elemental analysis for $C_{68}H_{109}N_3O_{12}S$: | | | | |
|---|---|---|---|---|
| Calcd.<br>Found | C, 68.48;<br>C, 68.31; | H, 9.21;<br>H, 9.25; | N, 3.52;<br>N, 3.72; | S, 2.69<br>S, 2.48 |

Compound HGCP-3: $[\alpha]_D^{24}$ - 16.4° (c = 0.53, in chloroform)

| Elemental analysis for $C_{53}H_{99}N_3O_{10}S \cdot 0.8H_2O$: | | | | |
|---|---|---|---|---|
| Calcd.<br>Found | C, 64.64;<br>C, 64.62; | H, 10.30;<br>H, 10.25; | N, 4.27;<br>N, 4.08; | S, 3.26<br>S, 3.22 |

Reference Example 12

Production of (2R,6R)-2-Fmoc-amino-6,7-bis(PamO)-4-THT-Gly-Glu($O^tBu$)-Glu($O^tBu$)-$O^tBu$ (FmocGCP-4) and (2R,6R)-2-amino-6,7-bis(PamO)-4-THT-Gly-Glu($O^tBu$)-Glu($O^tBu$)-$O^tBu$ (HGCP-4)

In substantially the same manner as in Reference Example 9, (2R,6R)-2-Fmoc-amino-6,7-bis(PamO)-4-THT-Gly-Glu($O^tBu$)-Glu($O^tBu$)-$O^tBu$ (FmocGCP-4) and (2R,6R)-2-amino-6,7-bis(PamO)-4-THT-Gly-Glu-($O^tBu$)-Glu($O^tBu$)-$O^tBu$ (HGCP-4) were produced.

| Materials (g) | Reaction Conditions | | Products (g) |
|---|---|---|---|
| a) FmocGC-1 (0.95) | HP-4<br>HONB<br>DIC<br>DMF<br>20 °C | 590 mg<br>210 mg<br>185 µl<br>9.5 ml<br>16 h | FmocGCP-4 (1.22) |
| b) FmocGCP-4 (1.10) | Piperidine<br>DCM<br>20 °C<br>Silica gel (chloroform-methanol) 97:3 | 1.1 ml<br>9.9 ml<br>1 h | HGCP-4 (0.97) |

Compound FmocGCP-4: $[\alpha]_D^{24}$ - 7.0° (c = 0.50, in chloroform)

| Elemental analysis for $C_{77}H_{124}N_4O_{15}S$: | | | | |
|---|---|---|---|---|
| Calcd.<br>Found | C, 67.12;<br>C, 67.16; | H, 9.07;<br>H, 9.05; | N, 4.07;<br>N, 4.10; | S, 2.33<br>S, 2.40 |

Compound HGCP-4: $[\alpha]_D^{24}$ - 10.6° (c = 0.50, in chloroform)

| Elemental analysis for $C_{62}H_{114}N_4O_{13}S \cdot 0.5H_2O$: | | | | |
|---|---|---|---|---|
| Calcd.<br>Found | C, 63.94;<br>C, 63.91; | H, 9.95;<br>H, 9.80; | N, 4.81;<br>N, 4.74; | S, 2.75<br>S, 2.71 |

Reference Example 13

Production of (2R,6R)-2-Fmoc-amino-6,7-bis(PamO)-4-THT-Glu(O$^t$Bu)-Gly-Glu(O$^t$Bu)-O$^t$Bu (FmocGCP-5) and (2R,6R)-2-amino-6,7-bis(PamO)-4-THT-Glu(O$^t$Bu)-Gly-Glu(O$^t$Bu)-O$^t$Bu (HGCP-5)

In substantially the same manner as in Reference Example 9, (2R,6R)-2-Fmoc-amino-6,7-bis(PamO)-4-THT-Glu(O$^t$Bu)-Gly-Glu(O$^t$Bu)-O$^t$Bu (FmocGCP-5) and (2R,6R)-2-amino-6,7-bis(PamO)-4-THT-Glu(O$^t$Bu)-Gly-Glu(O$^t$Bu)-O$^t$Bu (HGCP-5) were produced.

| Materials (g) | Reaction Conditions | | Products (g) |
|---|---|---|---|
| a) FmocGC-1 (1.24) | HP-5<br>HONB<br>DIC<br>DMF<br>20°C | 770 mg<br>274 mg<br>241 µl<br>12.0 ml<br>16 h | FmocGCP-5 (1.63) |
| b) FmocGCP-5 (1.50) | Piperidine<br>DCM<br>20°C<br>Silica gel (chloroform-methanol) 97:3 | 1.5 ml<br>13.5 ml<br>1 h | HGCP-5 (1.34) |

Compound FmocGCP-5: $[\alpha]_D^{24}$ - 1.7° (c = 0.51, in chloroform)

| Elemental analysis for $C_{77}H_{124}N_4O_{15}S$: | | | | |
|---|---|---|---|---|
| Calcd.<br>Found | C, 67.12;<br>C, 66.97; | H, 9.07;<br>H, 9.12; | N, 4.07;<br>N, 4.06; | S, 2.33<br>S, 2.25 |

Compound HGCP-5: $[\alpha]_D^{24}$ - 9.6° (c = 0.53, in chloroform)

| Elemental analysis for $C_{62}H_{114}N_4O_{13}S \cdot 1H_2O$: | | | | |
|---|---|---|---|---|
| Calcd.<br>Found | C, 63.45;<br>C, 63.31; | H, 9.96;<br>H, 9.81; | N, 4.77;<br>N, 4.82; | S, 2.73<br>S, 2.65 |

Reference Example 14

Production of (2R,6R)-2-Fmoc-amino-6,7-bis(PamO)-4-THT-Gly-Lys(Boc)-Gly-O$^t$Bu (FmocGCP-6) and (2R,6R)-2-amino-6,7-bis(pamO)-4-THT-Gly-Lys(Boc)-Gly-O$^t$Bu (HGCP-6)

In substantially the same manner as in Reference Example 9, (2R,6R)-2-Fmoc-amino-6,7-bis(PamO)-4-THT-Gly-Lys(Boc)-Gly-O$^t$Bu (FmocGCP-6) and (2R,6R)-2-amino-6,7-bis(PamO)-4-THT-Gly-Lys(Boc)-Gly-O$^t$Bu (HGCP-6) were produced.

| Materials (g) | Reaction Conditions | | Products (g) |
|---|---|---|---|
| a) FmocGC-1 (1.97) | HP-6<br>HONB<br>DIC<br>DMF<br>20 °C | 1.01 g<br>435 mg<br>0.38 ml<br>30 ml<br>13 h | FmocGCP-6 (2.24) |
| b) FmocGCP-6 (2.00) | Piperidine<br>DCM<br>20 °C<br>Silica gel (chloroform-methanol) 50:1 | 2.0 ml<br>20 ml<br>1.5 h | HGCP-6 (1.23) |

Compound FmocGCP-6: $[\alpha]_D^{25}$ - 10.0° (c = 0.66, in chloroform)

| Elemental analysis for $C_{72}H_{117}N_5O_{13}S$: | | | | |
|---|---|---|---|---|
| Calcd.<br>Found | C, 66.89;<br>C, 67.10; | H, 9.12;<br>H, 9.37; | N, 5.42;<br>N, 5.18; | S, 2.48<br>S, 2.49 |

Compound HGCP-6: $[\alpha]_D^{25}$ - 15.2° (c = 0.56, in chloroform)

| Elemental analysis for $C_{57}H_{107}N_5O_{11}S$: | | | | |
|---|---|---|---|---|
| Calcd.<br>Found | C, 63.95;<br>C, 63.70; | H, 10.07;<br>H, 10.20; | N, 6.54;<br>N, 6.50; | S, 3.00<br>S, 2.99 |

Example 1

Production of (2R,6R)-2-(H-Gly-amino)-6,7-bis(PamO)-4-THT-Glu-Gly-D-Glu-OH (compound 1)

a) To a solution of HGCP-2 (900 mg) synthesized in Reference Example 10 and Boc-Gly-OH (150 mg) in DCM (10 ml) were added, under ice-cooling, HOBT (116 mg) and WSC (165 mg). The mixture was stirred at 20 °C for 20 hours. This reaction mixture was concentrated, which was then dissolved in ethyl acetate (100 ml). The solution was washed with 10% (w/v) aqueous solution of ammonium chloride, 2% (w/v) aqueous solution of sodium hydrogencarbonate and water successively. The ethyl acetate layer was dried over anhydrous sodium sulfate, followed by concentration. The residue was suspended in acetonitrile, followed by filtration to afford (2R,6R)-2-(Boc-Gly-amino)-6,7-bis(PamO)-4-THT-Glu(O$^t$Bu)-Gly-D-Glu(O$^t$Bu)-O$^t$Bu (compound 1a) as white powdery product (811 mg, yield 80%).
$[\alpha]_D^{25}$ - 8.5° (c = 0.59, in chloroform)

| Elemental analysis for $C_{69}H_{125}N_5O_{16}S$: | | | | |
|---|---|---|---|---|
| Calcd.<br>Found | C, 63.13;<br>C, 63.04; | H, 9.60;<br>H, 9.52; | N, 5.33;<br>N, 5.26; | S, 2.44<br>S, 2.14 |

b) The compound 1a (715 mg) was dissolved in TFA (8 ml), and the solution was left standing at 20 °C for 2 hours. The reaction mixture was concentrated, and the residue was suspended in acetonitrile, followed by filtration to afford (2R,6R)-2-(H-Gly-amino)-6,7-bis(PamO)-4-THT-Glu-Gly-D-Glu-OH (compound 1) as white powdery product (580 mg, yield 98%).
$[\alpha]_D^{25}$ - 17.7° (c = 0.69, in 5% TFA-chloroform)

| Elemental analysis for $C_{52}H_{93}N_5O_{14}S \cdot 2H_2O$: | | | | |
|---|---|---|---|---|
| Calcd. | C, 57.81; | H, 9.05; | N, 6.48; | S, 2.97 |
| Found | C, 57.66; | H, 9.22; | N, 6.23; | S, 2.72 |

Example 2

Production of (2R,6R)-2-(H-Glu-amino)-6,7-bis(PamO)-4-THT-Glu-Gly-D-Glu-OH (compound 2)

a) To a solution of HGCP-2 (2.50 g) synthesized in Reference Example 10 and Fmoc-Glu($O^tBu$)-OH (1.06 g) in DCM (25 ml), were added, under ice-cooling, HOBT (322 mg) and WSC (456 mg). The mixture was stirred at 20°C for 16 hours. This reaction mixture was concentrated, which was then dissolved in ethyl acetate (200 ml). The solution was washed with 10% (w/v) aqueous solution of ammonium chloride, 2% (w/v) aqueous solution of sodium hydrogencarbonate solution and water, successively. The ethyl acetate layer was dried over anhydrous sodium sulfate, followed by concentration. The residue was suspended in acetonitrile, followed by filtration to afford (2R,6R)-2-(Fmoc-Glu($O^tBu$)-amino)-6,7-bis(PamO)-4-THT-Glu($O^tBu$)-Gly-D-Glu($O^tBu$)-$O^tBu$ (compound 2a) as white powdery product (3.00 g, yield 89%).
$[\alpha]_D^{25}$ - 9.7° (c=0.74, in chloroform)

| Elemental analysis for $C_{86}H_{139}N_5O_{18}S \cdot H_2O$: | | | | |
|---|---|---|---|---|
| Calcd. | C, 65.33; | H, 8.99; | N, 4.43; | S, 2.03 |
| Found | C, 65.58; | H, 8.96; | N, 4.49; | S, 2.08 |

b) The compound 2a (2.90 g) was dissolved in DCM (20 ml), to which was added piperidine (2.9 ml). The mixture was stirred at 20°C for 1.5 hours. This reaction mixture was then concentrated. The concentrate was subjected to a silica-gel column chromatography, eluting with chloroform-methanol mixtures (50:1, 20:1), successively. Fractions containing the object compound were combined and concentrated to leave (2R,6R)-2-(H-Glu($O^tBu$)-amino)-6,7-bis(PamO)-4-THT-Glu($O^tBu$)-Gly-D-Glu($O^tBu$)-$O^tBu$ (compound 2b) as white powdery product (1.88 g, yield 76%).
$[\alpha]_D^{25}$ - 13.1° (c=0.56, in chloroform)

| Elemental analysis for $C_{71}H_{129}N_5O_{16}S \cdot 0.5H_2O$: | | | | |
|---|---|---|---|---|
| Calcd. | C, 63.17; | H, 9.71; | N, 5.19; | S, 2.38 |
| Found | C, 63.25; | H, 9.66; | N, 4.95; | S, 2.24 |

c) The compound 2b (760 mg) was dissolved in TFA (8 ml), and the solution was left to standing at 20°C for 2 hours. This reaction mixture was concentrated, and the residue was suspended in acetonitrile, followed by filtration to afford (2R,6R)-2-(H-Glu-amino)-6,7-bis(PamO)-4-THT-Glu-Gly-D-Glu-OH (compound 2) as white powdery product (612 mg, yield 97%).
$[\alpha]_D^{25}$ - 4.0° (c=0.56, in 5% TFA-chloroform)

| Elemental analysis for $C_{55}H_{97}N_5O_{16}S \cdot 2H_2O$: | | | | |
|---|---|---|---|---|
| Calcd. | C, 57.32; | H, 8.83; | N, 6.08; | S, 2.78 |
| Found | C, 57.44; | H, 8.24; | N, 5.84; | S, 2.81 |

Example 3

Production of (2R,6R)-2-(H-Gly-Glu-amino)-6,7-bis(PamO)-4-THT-Glu-Gly-D-Glu-OH (compound 3)

In substantially the same manner as in Example 1, (2R,6R)-2-(Boc-Gly-Glu-amino)-6,7-bis(PamO)-4-THT-Glu($O^tBu$)-Gly-D-Glu($O^tBu$)-$O^tBu$ (compound 3a) and (2R,6R)-2-(H-Gly-Glu-amino)-6,7-bis(PamO)-4-THT-Glu-Gly-D-Glu-OH (compound 3) were produced.

| Materials (g) | Reaction Conditions | | Products (g) |
|---|---|---|---|
| a) Boc-Gly-OH (0.13) | 2b<br>HOBT<br>WSC<br>DCM<br>20°C | 900 mg<br>99 mg<br>142 mg<br>10 ml<br>14 h | 3a (0.96) |
| b) 3a (0.73) | TFA<br>20°C | 8.0 ml<br>2 h | 3 (0.59) |

Compound 3a: $[\alpha]_D^{25}$ - 15.3° (c=0.57, in chloroform)

| Elemental analysis for $C_{78}H_{140}N_6O_{19}S \cdot 0.5H_2O$: | | | | |
|---|---|---|---|---|
| Calcd.<br>Found | C, 62.16;<br>C, 62.24; | H, 9.43;<br>H, 9.15; | N, 5.58;<br>N, 5.31; | S, 2.13<br>S, 2.06 |

Compound 3: $[\alpha]_D^{25}$ - 27.8° (c=0.59, in 5% TFA-chloroform)

| Elemental analysis for $C_{57}H_{106}N_6O_{20}S \cdot 0.5H_2O$: | | | | |
|---|---|---|---|---|
| Calcd.<br>Found | C, 55.37;<br>C, 55.28; | H, 8.72;<br>H, 8.35; | N, 6.80;<br>N, 6.46; | S, 2.59<br>S, 2.39 |

Example 4

Production of (2R,6R)-2-(H-Glu-Gly-amino)-6,7-bis(Pamo)-4-THT-Glu-Gly-D-Glu-OH (compound 4)

In substantially the same manner as in Example 2, (2R,6R)-2-(Fmoc-Gly-amino)-6,7-bis(PamO)-4-THT-Glu($O^tBu$)-Gly-D-Glu($O^tBu$)-$O^tBu$ (compound 4a), (2R,6R)-2-(H-Gly-amino)-6,7-bis(PamO)-4-THT-Glu($O^tBu$)-Gly-D-Glu($O^tBu$)-$O^tBu$ (compound 4b), (2R,6R)-2-(Fmoc-Glu($O^tBu$)-Gly-amino)-6,7-bis(PamO)-4-THT-Glu-($O^tBu$)-Gly-D-Glu($O^tBu$)-$O^tBu$(compound 4c), (2R,6R)-2-(H-Glu($O^tBu$)-Gly-amino)-6,7-bis(PamO)-4-THT-Glu-($O^tBu$)-Gly-D-Glu($O^tBu$)-$O^tBu$ (compound 4d) and (2R,6R)-2-(H-Glu-Gly-amino)-6,7-bis(PamO)-4-THT-Glu-Gly-D-Glu-OH (compound 4) were produced.

| | Materials (g) | Reaction Conditions | | Products (g) |
|---|---|---|---|---|
| a) | Fmoc-Gly-OH (0.57) | HGCP-2<br>HOBT<br>WSC<br>DCM<br>20°C | 2.00 g<br>257 mg<br>365 mg<br>20 ml<br>16 h | 4a (2.08) |
| b) | 4a (2.03) | Piperidine<br>DCM<br>20°C<br>Silica gel (chloroform-methanol) 50:1-20:1 | 2.0 ml<br>20 ml<br>1.5 h | 4b (1.63) |
| c) | Fmoc-Glu(O$^t$Bu)-OH (0.30) | 4b<br>HOBT<br>WSC<br>DCM<br>20°C | 0.75 g<br>92 mg<br>130 mg<br>8.0 ml<br>16 h | 4c (0.86) |
| d) | 4c (0.81) | Piperidine<br>DCM<br>20°C<br>Silica gel (chloroform-methanol) 50:1-10:1 | 0.8 ml<br>8.0 ml<br>1.5 h | 4d (0.68) |
| e) | 4d (0.60) | TFA<br>20°C | 6.0 ml<br>1.5 h | 4 (0.49) |

Compound 4a: $[\alpha]_D^{24}$ - 9.9° (c=0.53, in chloroform)

| Elemental analysis for $C_{79}H_{127}N_5O_{16}S$: | | | | |
|---|---|---|---|---|
| Calcd.<br>Found | C, 66.13;<br>C, 66.01; | H, 8.92;<br>H, 9.01; | N, 4.88;<br>N, 4.64; | S, 2.23<br>S, 2.26 |

Compound 4b: $[\alpha]_D^{24}$ - 11.2° (c=0.57, in chloroform)

| Elemental analysis for $C_{64}H_{117}N_5O_{14}S$: | | | | |
|---|---|---|---|---|
| Calcd.<br>Found | C, 63.39;<br>C, 63.17; | H, 9.72;<br>H, 10.02; | N, 5.78;<br>N, 5.51; | S, 2.64<br>S, 2.62 |

Compound 4c: $[\alpha]_D^{24}$ - 10.6° (c=0.54, in chloroform)

| Elemental analysis for $C_{88}H_{142}N_6O_{19}S$: | | | | |
|---|---|---|---|---|
| Calcd.<br>Found | C, 65.24;<br>C, 65.12; | H, 8.83;<br>H, 8.90; | N, 5.19;<br>N, 5.03; | S, 1.98<br>S, 2.03 |

Compound 4d: $[\alpha]_D^{24}$ - 11.7° (c=0.52, in chloroform)

| Elemental analysis for $C_{73}H_{132}N_6O_{17}S$: | | | | |
|---|---|---|---|---|
| Calcd.<br>Found | C, 62.72;<br>C, 62.62; | H, 9.52;<br>H, 9.70; | N, 6.01;<br>N, 5.87; | S, 2.29<br>S, 2.31 |

Compound 4: $[\alpha]_D^{24}$ - 9.1° (c=0.59, in 5% TFA-chloroform)

| Elemental analysis for $C_{57}H_{100}N_6O_{17}S \cdot 3H_2O$: | | | | |
|---|---|---|---|---|
| Calcd.<br>Found | C, 55.77;<br>C, 55.63; | H, 8.70;<br>H, 8.61; | N, 6.85;<br>N, 6.46; | S, 2.61<br>S, 2.78 |

Example 5

Production of (2R,6R)-2-(H-Tyr-amino)-6,7-bis(PamO)-4-THT-Glu-Gly-D-Glu-OH (compound 5)

In substantially the same manner as in Example 2, (2R,6R)-2-(Fmoc-Tyr($^t$Bu)-amino)-6,7-bis(PamO)-4-THT-Glu(O$^t$Bu)-Gly-D-Glu(O$^t$Bu)-O$^t$Bu (compound 5a), (2R,6R)-2-(H-Tyr($^t$Bu)-amino)-6,7-bis(PamO)-4-THT-Glu(O$^t$Bu)-Gly-D-Glu(O$^t$Bu)-O$^t$Bu (compound 5b) and (2R,6R)-2-(H-Tyr-amino)-6,7-bis(PamO)-4-THT-Glu-Gly-D-Glu-OH (compound 5) were produced.

| Materials (g) | Reaction Conditions | | Products (g) |
|---|---|---|---|
| a) Fmoc-Tyr($^t$Bu)-OH (0.53) | HGCP-2<br>HOBT<br>WSC<br>DCM<br>20°C | 1.2 g<br>154 mg<br>219 mg<br>20 ml<br>21 h | 5a (1.49) |
| b) 5a (1.44) | Piperidine<br>DCM<br>20°C<br>Silica gel (chloroform-methanol) 50:1-10:1 | 1.4 ml<br>14 ml<br>1.5 h | 5b (1.24) |
| c) 5b (1.17) | TFA<br>20°C | 11.7 ml<br>2 h | 5 (0.98) |

Compound 5a: $[\alpha]_D^{25}$ - 11.6° (c=0.59, in chloroform)

| Elemental analysis for $C_{90}H_{141}N_5O_{17}S \cdot 0.5H_2O$: | | | | |
|---|---|---|---|---|
| Calcd.<br>Found | C, 67.30;<br>C, 67.32; | H, 8.91;<br>H, 8.74; | N, 4.36;<br>N, 4.20; | S, 2.00<br>S, 2.03 |

Compound 5b: $[\alpha]_D^{25}$ - 25.0° (c=0.59, in chloroform)

| Elemental analysis for $C_{75}H_{131}N_5O_{15}S \cdot 0.5H_2O$: | | | | |
|---|---|---|---|---|
| Calcd.<br>Found | C, 65.09;<br>C, 65.25; | H, 9.61;<br>H, 9.68; | N, 5.06;<br>N, 5.07; | S, 2.32<br>S, 2.27 |

Compound 5: $[\alpha]_D^{25}$ + 7.0° (c=0.61, in 5% TFA-chloroform)

| Elemental analysis for $C_{59}H_{99}N_5O_{15}S \cdot 2.5H_2O$: | | | | |
|---|---|---|---|---|
| Calcd. | C, 59.27; | H, 8.77; | N, 5.86; | S, 2.68 |
| Found | C, 59.45; | H, 8.48; | N, 5.60; | S, 2.54 |

Example 6

Production of (2R,6R)-2-(H-Lys-amino)-6,7-bis(PamO)-4-THT-Glu-Gly-D-Glu-OH (compound 6)

In substantially the same manner as in Example 2, (2R,6R)-2-(Fmoc-Lys(Boc)-amino)-6,7-bis(Pamo)-4-THT-Glu(O$^t$Bu)-Gly-D-Glu(O$^t$Bu)-O$^t$Bu (compound 6a), (2R,6R)-2-(H-Lys(Boc)-amino)-6,7-bis(PamO)-4-THT-Glu(O$^t$Bu)-Gly-D-Glu(O$^t$Bu)-O$^t$Bu (compound 6b) and (2R,6R)-2-(H-Lys-amino)-6,7-bis(PamO)-4-THT-Glu-Gly-D-Glu-OH (compound 6) were produced.

| | Materials (g) | Reaction Conditions | | Products (g) |
|---|---|---|---|---|
| a) | Fmoc-Lys(Boc)-OH (0.53) | HGCP-2 g<br>HOBT mg<br>WSC mg<br>DCM ml<br>20°C | 1.2<br>154<br>219<br>15<br>22 h | 6a (1.47) |
| b) | 6a (1.42) | Piperidine ml DCM ml<br>20°C h<br>Silica gel (chloroform-methanol) 50:1-20:1 | 1.4<br>14<br>1.5 | 6b (1.13) |
| c) | 6b (1.05) | TFA ml<br>20°C | 10<br>2 h | 6 (0.77) |

Compound 6a: $[\alpha]_D^{24}$ - 9.7° (c = 0.52, in chloroform)

| Elemental analysis for $C_{88}H_{144}N_6O_{18}S$: | | | |
|---|---|---|---|
| Calcd. | C, 65.81; | H, 9.04; | N, 5.23 |
| Found | C, 65.60; | H, 8.87; | N, 5.20 |

Compound 6b: $[\alpha]_D^{24}$ - 13.4° (c = 0.69, in chloroform)

| Elemental analysis for $C_{73}H_{134}N_6O_{16}S$: | | | | |
|---|---|---|---|---|
| Calcd. | C, 63.36; | H, 9.76; | N, 6.07; | S, 2.32 |
| Found | C, 63.22; | H, 9.89; | N, 5.90; | S, 2.31 |

Compound 6: $[\alpha]_D^{24}$ + 0.9° (c = 0.80, in 5% TFA-chloroform)

| Elemental analysis for $C_{56}H_{102}N_6O_{14}S \cdot 1.5H_2O$: | | | | |
|---|---|---|---|---|
| Calcd.<br>Found | C, 58.87;<br>C, 58.91; | H, 9.26;<br>H, 9.53; | N, 7.36;<br>N, 7.39; | S, 2.81<br>S, 2.76 |

Example 7

Production of (2R,6R)-2-(H-β-Ala-amino)-6,7-bis(PamO)-4-THT-Glu-Gly-D-Glu-OH (compound 7)

In substantially the same manner as in Example 1, (2R,6R)-2-(Boc-β-Ala-amino)-6,7-bis(PamO)-4-THT-Glu(O$^t$Bu)-Gly-D-Glu(O$^t$Bu)-O$^t$Bu (compound 7a) and (2R,6R)-2-(H-β-Ala-amino)-6,7-bis(PamO)-4-THT-Glu-Gly-D-Glu-OH (compound 7) were produced.

| Materials (g) | Reaction Conditions | | Products(g) |
|---|---|---|---|
| a) Boc-β-Ala-OH (0.14) | HGCP-2<br>HOBT<br>WSC<br>DCM<br>20 °C | 800 mg<br>103 mg<br>146 mg<br>8.0 ml<br>15 h | 7a (0.80) |
| b) 7a (0.75) | TFA<br>20 °C | 7.5 ml<br>2 h | 7 (0.59) |

Compound 7a: $[\alpha]_D^{24}$ - 8.2° (c=0.57, in chloroform)

| Elemental analysis for $C_{70}H_{127}N_5O_{16}S$: | | | | |
|---|---|---|---|---|
| Calcd.<br>Found | C, 63.37;<br>C, 63.37; | H, 9.65;<br>H, 9.43; | N, 5.28;<br>N, 5.17; | S, 2.42<br>S, 2.45 |

Compound 7: $[\alpha]_D^{24}$ - 14.3° (c=0.60, in 5% TFA-chloroform)

| Elemental analysis for $C_{53}H_{95}N_5O_{14}S \cdot 2H_2O$: | | | | |
|---|---|---|---|---|
| Calcd.<br>Found | C, 58.16;<br>C, 58.17; | H, 9.12;<br>H, 9.06; | N, 6.40;<br>N, 6.56; | S, 2.93<br>S, 2.84 |

Example 8

Production of (2R,6R)-2-(H-Gly-amino)-6,7-bis(PamO)-4-THT-Glu-Gly-Glu-OH (compound 8)

In substantially the same manner as in Example 1, (2R,6R)-2-(Boc-Gly-amino)-6,7-bis(PamO)-4-THT-Glu(O$^t$Bu)-Gly-Glu(O$^t$Bu)-O$^t$Bu (compound 8a) and (2R,6R)-2-(H-Gly-amino)-6,7-bis(PamO)-4-THT-Glu-Gly-Glu-OH (compound 8) were produced.

| Materials (g) | Reaction Conditions | | Products (g) |
|---|---|---|---|
| a) Boc-Gly-OH (0.15) | HGCP-5<br>HOBT<br>WSC<br>DCM<br>20 °C | 900 mg<br>116 mg<br>165 mg<br>10 ml<br>16 h | 8a (0.74) |
| b) 8a (0.65) | TFA<br>20 °C | 6.5 ml<br>2 h | 8 (0.53) |

Compound 8a: $[\alpha]_D^{24}$ - 9.3 ° (c = 0.61, in chloroform)

| Elemental analysis for $C_{69}H_{125}N_5O_{16}S$: | | | | |
|---|---|---|---|---|
| Calcd.<br>Found | C, 63.13;<br>C, 63.08; | H, 9.60;<br>H, 9.31; | N, 5.34;<br>N, 5.20; | S, 2.44<br>S, 2.48 |

Compound 8: $[\alpha]_D^{24}$ - 15.1 ° (c = 0.60, in 5% TFA-chloroform)

| Elemental analysis for $C_{52}H_{93}N_5O_{14}S \cdot 1.5H_2O$: | | | | |
|---|---|---|---|---|
| Calcd.<br>Found | C, 58.29;<br>C, 58.25; | H, 9.03;<br>H, 8.88; | N, 6.54;<br>N, 6.42; | S, 2.99<br>S, 2.82 |

Example 9

Production of (2R,6R)-2-(H-Gly-amino)-6,7-bis(PamO)-4-THT-Gly-Glu-Glu-OH (compound 9)

In substantially the same manner as in Example 1, (2R,6R)-2-(Boc-Gly-amino)-6,7-bis(PamO)-4-THT-Gly-Glu(O$^t$Bu)-Glu(O$^t$Bu)-O$^t$Bu (compound 9a) and (2R,6R)-2-(H-Gly-amino)-6,7-bis(PamO)-4-THT-Gly-Glu-Glu-OH (compound 9) were produced.

| Materials (g) | Reaction Conditions | | Products (g) |
|---|---|---|---|
| a) Boc-Gly-OH (0.13) | HGCP-4<br>HOBT<br>WSC<br>DCM<br>20 °C | 800 mg<br>103 mg<br>146 mg<br>8.0 ml<br>18 h | 9a (0.82) |
| b) 9a (0.74) | TFA<br>20 °C | 7.1 ml<br>2 h | 9 (0.56) |

Compound 9a: $[\alpha]_D^{24}$ - 7.2 ° (c = 0.70, in chloroform)

| Elemental analysis for $C_{69}H_{125}N_5O_{16}S$: | | | | |
|---|---|---|---|---|
| Calcd.<br>Found | C, 63.13;<br>C, 63.05; | H, 9.60;<br>H, 9.46; | N, 5.34;<br>N, 5.34; | S, 2.44<br>S, 2.38 |

Compound 9: $[\alpha]_D^{24}$ - 14.9 ° (c = 0.56, in 5% TFA-chloroform)

| Elemental analysis for $C_{52}H_{93}N_5O_{14}S \cdot 2H_2O$: | | | | |
|---|---|---|---|---|
| Calcd.<br>Found | C, 57.81;<br>C, 57.68; | H, 9.05;<br>H, 8.78; | N, 6.48;<br>N, 6.31; | S, 2.97<br>S, 2.80 |

Example 10

Production of (2R,6R)-2-(H-Glu-Gly-amino)-6,7-bis(PamO)-4-THT-Gly-Glu-Glu-OH (compound 10)

In substantially the same manner as in Example 2, (2R,6R)-2-(Fmoc-Gly-amino)-6,7-bis(PamO)-4-THT-Gly-Glu($O^tBu$)-Glu($O^tBu$)-$O^tBu$ (compound 10a), (2R,6R)-2-(H-Gly-amino)-6,7-bis(PamO)-4-THT-Gly-Glu-($O^tBu$)-Glu($O^tBu$)-$O^tBu$ (compound 10b), (2R,6R)-2-(Fmoc-Glu($O^tBu$)-Gly-amino)-6,7-bis(PamO)-4-THT-Gly-Glu($O^tBu$)-Glu($O^tBu$)-$O^tBu$ (compound 10c), (2R,6R)-2-(H-Glu($O^tBu$)-Gly-amino)-6,7-bis(PamO)-4-THT-Gly-Glu($O^tBu$)-Glu($O^tBu$)-$O^tBu$ (compound 10d) and (2R,6R)-2-(H-Glu-Gly-amino)-6,7-bis(PamO)-4-THT-Gly-Glu-Glu-OH (compound 10) were produced.

| Materials (g) | Reaction Conditions | | Products (g) |
|---|---|---|---|
| a) Fmoc-Gly-OH (1.41) | HGCP-4<br>HOBT<br>WSC<br>DCM<br>20 ° C | 5.00 g<br>643 mg<br>912 mg<br>50 ml<br>14 h | 10a (4.92) |
| b) 10a (4.70) | Piperidine<br>DCM<br>20 ° C<br>Silica gel (chloroform-methanol) 50:1-20:1 | 5.0 ml<br>50 ml<br>2 h | 10b (2.71) |
| c) Fmoc-Glu($O^tBu$)-OH (0.56) | 10b<br>HOBT<br>WSC<br>DCM<br>20 ° C | 2.6 g<br>312 mg<br>442 mg<br>50 ml<br>15 h | 10c (3.07) |
| d) 10c (2.89) | Piperidine<br>DCM<br>20 ° C<br>Silica gel (chloroform-methanol) 50:1-20:1 | 2.9 ml<br>30 ml<br>1.5 h | 10d (2.07) |
| e) 10d (0.59) | TFA<br>20 ° C | 6.0 ml<br>2 h | 10 (0.49) |

Compound 10a: $[\alpha]_D^{24}$ - 8.5 ° (c = 0.91, in chloroform)

| Elemental analysis for $C_{79}H_{127}N_5O_{16}S$: | | | | |
|---|---|---|---|---|
| Calcd.<br>Found | C, 66.13;<br>C, 65.99; | H, 8.92;<br>H, 8.93; | N, 4.88;<br>N, 5.00; | S, 2.23<br>S, 2.60 |

Compound 10b: $[\alpha]_D^{24}$ - 6.1 ° (c = 0.81, in chloroform)

| Elemental analysis for $C_{64}H_{117}N_5O_{14}S \cdot 0.5H_2O$: | | | | |
|---|---|---|---|---|
| Calcd.<br>Found | C, 62.92;<br>C, 63.01; | H, 9.74;<br>H, 9.78; | N, 5.73;<br>N, 6.00; | S, 2.62<br>S, 2.54 |

Compound 10c: $[\alpha]_D^{24}$ - 7.9° (c = 0.67, in chloroform)

| Elemental analysis for $C_{88}H_{142}N_6O_{19}S \cdot 0.5H_2O$: | | | | |
|---|---|---|---|---|
| Calcd.<br>Found | C, 64.88;<br>C, 64.87; | H, 8.85;<br>H, 8.76; | N, 5.16;<br>N, 5.27; | S, 1.97<br>S, 1.99 |

Compound 10d: $[\alpha]_D^{24}$ - 12.8° (c = 0.89, in chloroform)

| Elemental analysis for $C_{73}H_{132}N_6O_{17}S \cdot 0.5H_2O$: | | | | |
|---|---|---|---|---|
| Calcd.<br>Found | C, 62.32;<br>C, 62.27; | H, 9.53;<br>H, 9.44; | N, 5.97;<br>N, 6.11; | S, 2.28<br>S, 2.23 |

Compound 10: $[\alpha]_D^{24}$ - 2.4° (c = 0.76, in 5% TFA-chloroform)

| Elemental analysis for $C_{57}H_{100}N_6O_{17}S \cdot 4H_2O$: | | | | |
|---|---|---|---|---|
| Calcd.<br>Found | C, 54.97;<br>C, 54.84; | H, 8.74;<br>H, 8.59; | N, 6.75;<br>N, 6.60; | S, 2.57<br>S, 2.55 |

Example 11

Production of (2R,6R)-2-(H-Glu-Glu-Gly-amino)-6,7-bis(PamO)-4-THT-Gly-Glu-Glu-OH (compound 11)

In substantially the same manner as in Example 2, (2R,6R)-2-(Fmoc-Glu($O^tBu$)-Glu($O^tBu$)-Gly-amino)-6,7-bis(PamO)-4-THT-Gly-Glu($O^tBu$)-Glu($O^tBu$)-$O^tBu$ (compound 11a), (2R,6R)-2-(H-Glu($O^tBu$)-Glu($O^tBu$)-Gly-amino)-6,7-bis(PamO)-4-THT-Gly-Glu($O^tBu$)-Glu($O^tBu$)-$O^tBu$ (compound 11b) and (2R,6R)-2-(H-Glu-Glu-Gly-amino)-6,7-bis(PamO)-4-THT-Gly-Glu-Glu-OH (compound 11) were produced.

| Materials (g) | Reaction Conditions | | Products (g) |
|---|---|---|---|
| a) Fmoc-Glu($O^tBu$)-OH (0.35) | 10d<br>HOBT<br>WSC<br>DCM<br>20 °C | 1.0 g<br>106 mg<br>151 mg<br>25 ml<br>15 h | 11a (1.20) |
| b) 11a (1.00) | Piperidine<br>DCM<br>20 °C<br>Silica gel (chloroform-methanol) 50:1 | 1.0 ml<br>20 ml<br>2 h | 11b (0.67) |
| c) 11b (0.57) | TFA<br>20 °C | 5.7 ml<br>2 h | 11 (0.47) |

Compound 11a: $[\alpha]_D^{24}$ - 11.1° (c = 0.60, in chloroform)

| Elemental analysis for $C_{91}H_{157}N_7O_{22}S \cdot 0.5H_2O$: | | | | |
|---|---|---|---|---|
| Calcd.<br>Found | C, 64.21;<br>C, 64.00; | H, 8.78;<br>H, 8.66; | N, 5.40;<br>N, 5.24; | S, 1.77<br>S, 1.63 |

Compound 11b: $[\alpha]_D^{24}$ - 10.1° (c = 0.55, in chloroform)

| Elemental analysis for $C_{82}H_{147}N_7O_{20}S$: | | | | |
|---|---|---|---|---|
| Calcd.<br>Found | C, 62.21;<br>C, 62.03; | H, 9.36;<br>H, 9.57; | N, 6.19;<br>N, 6.10; | S, 2.03<br>S, 2.07 |

Compound 11: $[\alpha]_D^{24}$ - 3.8° (c=0.56, in 5% TFA-chloroform)

| Elemental analysis for $C_{62}H_{107}N_7O_{20}S \cdot 2H_2O$: | | | | |
|---|---|---|---|---|
| Calcd.<br>Found | C, 55.63;<br>C, 55.58; | H, 8.36;<br>H, 8.12; | N, 7.32;<br>N, 7.09; | S, 2.40<br>S, 2.39 |

Example 12

Production of (2R,6R)-2-(H-Glu-amino)-6,7-bis(PamO)-4-THT-Gly-D-Glu-OH (compound 12)

In substantially the same manner as in Example 2, (2R,6R)-2-(Fmoc-Glu(O$^t$Bu)-amino)-6,7-bis(PamO)-4-THT-Gly-D-Glu(O$^t$Bu)-O$^t$Bu (compound 12a), (2R,6R)-2-(H-Glu(O$^t$Bu)-amino)-6,7-bis(PamO)-4-THT-Gly-D-Glu(O$^t$Bu)-O$^t$Bu (compound 12b) and (2R,6R)-2-(H-Glu-amino)-6,7-bis(PamO)-4-THT-Gly-D-Glu-OH (compound 12) were produced.

| Materials (g) | Reaction Conditions | | Products (g) |
|---|---|---|---|
| a) Fmoc-Glu(O$^t$Bu)-OH (0.60) | HGCP-3<br>HOBT<br>WSC<br>DCM<br>20°C | 1.2 g<br>184 mg<br>261 mg<br>25 ml<br>20 h | 12a (1.22) |
| b) 12a (1.07) | Piperidine<br>DCM<br>20°C<br>Silica gel (chloroform-methanol) 50:1 | 1.1 ml<br>10 ml<br>1.5 h | 12b (0.70) |
| c) 12b (0.54) | TFA<br>20°C | 5.4 ml<br>1.5 h | 12 (0.46) |

Compound 12a: $[\alpha]_D^{24}$ - 16.3° (c=0.62, in chloroform)

| Elemental analysis for $C_{77}H_{124}N_4O_{15}S$: | | | | |
|---|---|---|---|---|
| Calcd.<br>Found | C, 67.12;<br>C, 66.97; | H, 9.07;<br>H, 8.94; | N, 4.07;<br>N, 4.07; | S, 2.33<br>S, 2.33 |

Compound 12b: $[\alpha]_D^{24}$ - 21.2° (c=0.55, in chloroform)

| Elemental analysis for $C_{62}H_{114}N_4O_{13}S$: | | | | |
|---|---|---|---|---|
| Calcd.<br>Found | C, 64.44;<br>C, 64.13; | H, 9.94;<br>H, 9.96; | N, 4.85;<br>N, 4.81; | S, 2.77<br>S, 2.73 |

Compound 12: $[\alpha]_D^{24}$ + 4.5° (c=0.41, in 5% TFA-chloroform)

| Elemental analysis for $C_{50}H_{90}N_4O_{13}S \cdot 4H_2O$: | | | | |
|---|---|---|---|---|
| Calcd.<br>Found | C, 56.69;<br>C, 56.47; | H, 9.32;<br>H, 8.87; | N, 5.29;<br>N, 5.11; | S, 3.03<br>S, 3.04 |

Example 13

Production of (2R,6R)-2-(H-β-Ala-amino)-6,7-bis(PamO)-4-THT-Gly-Gly-Gly-Glu-OH (compound 13)

To a solution of HGCP-1(43 mg) synthesized in Reference Example 9 and Boc-β-Ala-OH (9 mg) in DMF (1 ml) were added TEA (0.014 ml) and DEPC (10 mg). The mixture was stirred at 20 °C for 5 hours. This reaction mixture was added water, which was subjected to extraction with ethyl acetate. The ethyl acetate layer was washed with 5% (w/v) aqueous solution of citric acid, saturated aqueous solution of sodium hydrogencarbonate and saturated aqueous saline solution, successively. The ethyl acetate layer was dried over anhydrous sodium sulfate and the solvent was then distilled off. The residue was subjected to a silica-gel column chromatography (chloroform-methanol = 93:7) to give (2R,6R)-2-(Boc-β-Ala-amino)-6,7-bis-(PamO)-4-THT-Gly-Gly-Gly-Glu(O$^t$Bu)-O$^t$Bu (compound 13a) as a colorless wax-like product (25 mg, yield 50%).

IR (KBr) ν: 3290, 1735, 1630 $cm^{-1}$

$^1$H-NMR ($CDCl_3$) δ: 0.88 (6H, t, J = 6.6 Hz), 1.03-1.35 (48H,m), 1.41 (9H, s), 1.43 (9H, s), 1.47 (9H, s), 1.50-1.70 (4H, m), 1.70-3.60 (14H, m), 3.85-4.67 (11H, m), 6.50-8.30 (6H, m)

To this compound (13a) (25 mg) was added 4N HCl-ethyl acetate (4 ml). The mixture was stirred at 20 °C for 4 hours. The solvent was then distilled off to give compound 13 as a white powdery product (21 mg, yield 100%).

m.p.: 98-100 °C

IR (KBr) ν: 3400, 1735, 1655 $cm^{-1}$

$^1$H-NMR ($CDCl_3$) δ: 0.88 (6H, t, J = 6.9 Hz), 1.03-1.47 (48H,m), 1.47-1.72 (4H, m), 1.72-3.20 (16H, m), 3.75-4.60 (10H, m), 5.10-5.25 (1H, m)

Example 14

Production of (2R,6R)-2-(H-Gly-amino)-6,7-bis(PamO)-4-THT-Gly-Lys-Gly-OH (compound 14)

In substantially the same manner as in Example 1, (2R,6R)-2-(Boc-Gly-amino)-6,7-bis(PamO)-4-THT-Gly-Lys(Boc)-Gly-O$^t$Bu (compound 14a) and (2R,6R)-2-(H-Gly-amino)-6,7-bis(PamO)-4-THT-Gly-Lys-Gly-OH (compound 14) were produced.

| Materials (mg) | Reaction Conditions | | Products (mg) |
|---|---|---|---|
| a) Boc-Gly-OH (45) | HGCP-6<br>HOBT<br>WSC<br>DCM<br>20 °C | 250 mg<br>35 mg<br>49 mg<br>10 ml<br>14 h | 14a (253) |
| b) 14a (150) | TFA<br>20 °C | 1.5 ml<br>2 h | 14 (140) |

Compound 14a: $[\alpha]_D^{25}$ - 16.2 ° (c = 0.55, in chloroform)

| Elemental analysis for $C_{64}H_{118}N_6O_{14}S \cdot 0.5H_2O$: | | | | |
|---|---|---|---|---|
| Calcd.<br>Found | C, 62.16;<br>C, 62.13; | H, 9.70;<br>H, 9.61; | N, 6.79;<br>N, 7.03; | S, 2.59<br>S, 2.59 |

Compound 14: $[\alpha]_D^{25}$ - 18.3 ° (c = 0.59, in chloroform)

| Elemental analysis for $C_{50}H_{94}N_6O_{10}S \cdot 2TFA \cdot 1.5H_2O$: | | | | |
|---|---|---|---|---|
| Calcd. | C, 52.88; | H, 8.14; | N, 6.85; | S, 2.61 |
| Found | C, 53.01; | H, 8.02; | N, 6.86; | S, 2.65 |

Example 15

Preparation of sodium salt of compound 1 (compound 15)

Compound 1 (450 mg) was dissolved in 5% (v/v) acetonitrile-0.5% (w/v) sodium hydrogen carbonate aqueous solution (225 ml) under heating. After adjusting pH of the solution at 9.5, the solution was subjected to a chromatography on Diaion HP-20 (45 ml, Mitsubishi Kasei Corp. Japan) which was previously swollen with water. The resion was washed with 5% (v/v) aqueous acetonitrile (260 ml), followed by development with 40% aqueous acetonitrile (250 ml). Eluent was concentrated and then freeze-dried to give a powdery product. The powdery product was suspended in acetone (5 ml). Insolubles were collected by filtration to give disodium salt of compound 1 (compound 15, 352 mg) as a white powder.

| Elemental analysis for $C_{52}H_{91}N_5O_{14}SNa_2 \cdot 2H_2O$: | | | | | |
|---|---|---|---|---|---|
| Calcd. | C, 55.55; | H, 8.56; | N, 6.23; | S, 2.85; | Na, 4.09 |
| Found | C, 55.27; | H, 8.56; | N, 6.13; | S, 3.35; | Na, 4.6 |

Example 16

Preparation of sodium salt of compound 2 (compound 16)

Compound 2 (400 mg) was dissolved in 0.5% (w/v) sodium hydrogen carbonate aqueous solution (200 ml) under heating. After adjusting pH of the solution at 9.5, the solution was subjected to a chromatography on Diaion HP-20 (80 ml, Mitsubishi Kasei Corp. Japan) which was previously swollen with water. The resion was washed with water (540 ml), followed by development with 40% aqueous acetonitrile (240 ml). Eluent was concentrated and then freeze-dried to give a powdery product. The powdery product was suspended in acetonitrile (5 ml). Insolubles were collected by filtration to give trisodium salt of compound 2 (compound 16, 352 mg) as a white powder.

| Elemental analysis for $C_{55}H_{94}N_5O_{16}SNa_3 \cdot 3H_2O$: | | | | | |
|---|---|---|---|---|---|
| Calcd. | C, 53.43; | H, 8.15; | N, 5.66; | S, 2.59; | Na, 5.58 |
| Found | C, 53.39; | H, 8.08; | N, 5.56; | S, 2.57; | Na, 5.5 |

Example 17

Preparation of sodium salt of compound 4 (compound 17)

Compound 4 (300 mg) was dissolved in 0.5% (w/v) sodium hydrogen carbonate aqueous solution (150 ml) under heating. After adjusting pH of the solution at 9.5, the solution was subjected to a chromatography on Diaion HP-20 (60 ml, Mitsubishi Kasei Corp. Japan) which was previously swollen with water. The resion was washed with water (480 ml), followed by development with 40% (v/v) aqueous acetonitrile (180 ml). Eluent was concentrated and then freeze-dried to give a powdery product. The powdery product was suspended in acetonitrile (40 ml). Insolubles were collected by filtration to give trisodium salt of compound 4 (compound 17, 252 mg) as a white powder.

| Elemental analysis for $C_{57}H_{97}N_6O_{17}SNa_3 \cdot 2H_2O$: | | | | | |
|---|---|---|---|---|---|
| Calcd. | C, 53.68; | H, 7.98; | N, 6.59; | S, 2.51; | Na, 5.41 |
| Found | C, 53.43; | H, 7.99; | N, 6.52; | S, 3.03; | Na, 5.3 |

Example 18

Preparation of sodium salt of compound 6 (compound 18)

Compound 6 (500 mg) was dissolved in 0.5% (w/v) sodium hydrogencarbonate aqueous solution (250 ml) under heating. After adjusting pH of the solution at 9.5, the solution was subjected to a chromatography on Diaion HP-20 (100 ml, Mitsubishi Kasei Corp. Japan) which was previously swollen with water. The resion was washed with water (800 ml), followed by development with 40% aqueous acetonitrile (400 ml) and 60% (v/v) aqueous acetonitrile (200 ml). Eluent was concentrated and then freeze-dried to give a powdery product. The powdery product was suspended in acetonitrile (40 ml). Insolubles were collected by filtration to give disodium salt of compound 6 (compound 18, 277 mg) as a white powder.

| Elemental analysis for $C_{56}H_{100}N_6O_{14}SNa_2 \cdot 2H_2O$: | | | | | |
|---|---|---|---|---|---|
| Calcd. | C, 56.26; | H, 8.77; | N, 7.03; | S, 2.68; | Na, 3.85 |
| Found | C, 56.10; | H, 8.61; | N, 7.01; | S, 2.30; | Na, 3.5 |

Structural formulae of the compounds obtained in the above examples are shown in Table 1.

[Table 1]

| Compound No. | Example No. | Structural Formulae |
|---|---|---|
| 1 | 1 | (2R,6R)-2-(H-Gly-amino)-6,7-bis(PamO)-4-THT-Glu-Gly-D-Glu-OH |
| 2 | 2 | (2R,6R)-2-(H-Glu-amino)-6,7-bis(PamO)-4-THT-Glu-Gly-D-Glu-OH |
| 3 | 3 | (2R,6R)-2-(H-Gly-Glu-amino)-6,7-bis(PamO)-4-THT-Glu-Gly-D-Glu-OH |
| 4 | 4 | (2R,6R)-2-(H-Glu-Gly-amino)-6,7-bis-(PamO)-4-THT-Glu-Gly-D-Glu-OH |
| 5 | 5 | (2R,6R)-2-(H-Tyr-amino)-6,7-bis(PamO)-4-THT-Glu-Gly-D-Glu-OH |
| 6 | 6 | (2R,6R)-2-(H-Lys-amino)-6,7-bis(PamO)-4-THT-Glu-Gly-D-Glu-OH |
| 7 | 7 | (2R,6R)-2-(H-β-Ala-amino)-6,7-bis(PamO)-4-THT-Glu-Gly-D-Glu-OH |
| 8 | 8 | (2R,6R)-2-(H-Gly-amino)-6,7-bis(PamO)-4-THT-Glu-Gly-Glu-OH |
| 9 | 9 | (2R,6R)-2-(H-Gly-amino)-6,7-bis(PamO)-4-THT-Gly-Glu-Glu-OH |
| 10 | 10 | (2R,6R)-2-(H-Glu-Gly-amino)-6,7-bis(PamO)-4-THT-Gly-Glu-Glu-OH |
| 11 | 11 | (2R,6R)-2-(H-Glu-Glu-Gly-amino)-6,7-bis(PamO)-4-THT-Gly-Glu-Glu-OH |
| 12 | 12 | (2R,6R)-2-(H-Glu-amino)-6,7-bis(PamO)-4-THT-Gly-D-Glu-OH |
| 13 | 13 | (2R,6R)-2-(H-β-Ala-amino)-6,7-bis(PamO)-4-THT-Gly-Gly-Gly-Glu-OH |
| 14 | 14 | (2R,6R)-2-(H-Gly-amino)-6,7-bis(PamO)-4 THT-Gly-Lys-Gly-OH·2TFA |
| 15 | 15 | (2R,6R)-2-(H-Gly-amino)-6,7-bis(PamO)-4-THT-Glu-Gly-D-Glu-OH·2Na |
| 16 | 16 | (2R,6R)-2-(H-Glu-amino)-6,7-bis(PamO)-4- |

| | | |
|---|---|---|
| | | THT-Glu-Gly-D-Glu-OH·3Na |
| 17 | 17 | (2R,6R)-2-(H-Glu-Gly-amino)-6,7--bis(PamO)-4-THT-Glu-Gly-D-Glu-OH·3Na |
| 18 | 18 | (2R,6R)-2-(H-Lys-amino)-6,7-bis(PamO)-4-THT-Glu-Gly-D-Glu-OH·2Na |

Biological activities of the compound (I) are described below.

Experimental Example 1

Actions of compound (I) produced in the foregoing Examples on enhancing proliferation of bone marrow cells of mice are shown in Table 2.

Method of determination:

To a RPMI 1640 culture medium [Bio-Wittaker Inc. (hereinafter abbreviated as BW), U.S.A.] containing 2 x $10^6$/ml of bone marrow cells of BALB/c mice, 2 mM of L-glutamine, 20 μg/ml of gentamicin (Flow Laboratories, Inc., Scotland), 10% fetal calf serum (BW, U.S.A.) was added a test compound in an adequate concentration, which was incubated at 37°C for 3 days in 5% carbon dioxide in air followed by determination of proliferation of the bone marrow cells by the MTT reduction method [Tada et al., Journal of Immunological Methods, Vol. 93, p.157, 1986].

Table 2

| Actions on enhancing proliferation of bone marrow cells of mice | |
|---|---|
| Compound No. | Minimal Effective Concentration (MEC, ng/ml)*1 |
| 1 | 6.25 |
| 5 | 6.25 |

*1 Assuming that proliferation in the group, to which no test compound was added, was 1, concentrations at which 1.3 or more times as much proliferation was observed were taken.

Experimental Example 2

Actions of compound (I) on enhancing the number of leukocytes in mice are shown in Table 3.

Method of determination:

Six weeks old female CDF1/Crj mice (5 animals per group) were subjected to the experiment. Each animal was orally administered with cyclophosphamide dissolved in physiological saline solution at a dose of 150 mg/kg. From the next day, each animal was administered subcutaneously with the compound suspended in 5% glucose at the following dosage for five days once a day. On the next day after completion of the administration, about 100 μl of peripheral blood was collected from orbital vein using an EDTA-treated glass capillary. Then, the number of leukocytes was counted using an automatic cell analyzer (Sysmex K-2000, Toa Medical Electronics, Japan).

Table 3

| Actions on enhancing the number of leukocytes | | |
|---|---|---|
| Compound No. | Dosage (mg/kg/day) | Number of leukocytes *1 |
| 1 | 0.50 | 137 |
| 4 | 0.13 | 108 |

*1 The value is shown by percentage relative to the number of leukocyte (assumed as 100%) of the mouse orally administered with physiological saline solution, in place of cyclophosphamide, at a dose of 0.2 ml relative to 20 g of body weight, then subcutaneously administered, from the next days of the oral administration, with 5% glucose once a day for 5 days. Incidentally, the average value and standard deviation of leukocyte number of mice orally administered with cyclophosphamide at a dose of 150 mg/kg, then from the next day, subcutaneously administered with 0.2 ml of 5% glucose relative to 20 g of body weight once a day for 5 days was 34.6 ± 9.5% throughout the experiment.

Experimental Example 3

Action of compound (I) on enhancing development of acetylcholine esterase (hereinafter abbreviated as AchE) positive cells are shown in Table 4.

Method of determination:

The AchE activity was studied according to a fluorescence method (Ishibashi et al., Proceedings of the National Academy of Science U.S.A., Vol. 86, pp. 5953 to 5957, 1989).

Non adherent bone marrow cells of BALB/c mice (1 x $10^6$ cells/ml) were suspended in Iscove's modification of Dulbecco's medium (Gibco BRL Inc., U.S.A.) containing 1% Neutridoma-SP (Boheringer Mannhaim, Germany). Twenty-five $\mu$l of a test compound solution in an adequate concentration was inoculated in a 96-well plate. To each of these solutions in the well was added 100 $\mu$l of the non adherent bone marrow cells suspension. After incubation at 37 °C for 5 days, 25 $\mu$l of 6% glutaraldehyde aqueous solution was added to the well. After standing at 4 °C for 30 minutes the mixture was centrifuged (850 x g) at 5 °C for 5 minutes and the supernatant was removed. After washing the deposit in the well with 100 $\mu$l of phosphate buffered saline, to the deposit was added 100 $\mu$l of buffer (pH 7.5) containing 0.2% polyoxyethylene-10-octylphenyl ether (POPE), 1 mM ethylenediaminetetraacetate (EDTA), 0.12 M sodium chloride and 50 mM N-2-hydroxyethylpiperadine-N'-2-ethansulfonic acid (HEPES). To this was added 10 $\mu$l of actylthiocholine iodide aqueous solution (concentration: 1.6 mg/ml). After incubation at 33 °C for 3 hours 10 $\mu$l of the resulting solution was separated. To this was added 10 $\mu$l of 0.4 mM 7-diethylamino-3-(4'-maleimidylphenyl)-4-methylcoumarin aqueous solution and 100 $\mu$l of buffer (pH 5.0) containing 0.2% POPE, 1 mM EDTA and 50 mM sodium acetate and fluorescence emission was determined on a fluorometer (excitation wave length: 365 nm, fluorescence wave length: 450 nm).

Table 4

| Actions on enhancing development of AchE Positive cells | | |
|---|---|---|
| Compound No. | Concentration (ng/ml) | Degree of AchE Positive Cell[a)] |
| 1 | 10 | 1.92 |

a) AchE activity in the group to which compound (I) is not added is shown as 1.

Experimental Example 4

Toxicity test

Intravenous administration of trisodium salt of compound 1 at 125 mg/kg caused no death in mice.

As in clear from the foregoing Experimental Examples, compound (I) or salt thereof has an activity of remarkably improving hematopoietic disorder and a low toxic potential, which can be utilized safely as a therapeutic or prophylactic agent of leukocytopenia caused by radiotherapy or chemotherapy of cancers in mammals, as a hematopoietic-stimulating agent in the case of bone marrow transplantation, as an immunopotentiator having leukocyte-increasing activity, and, further as a therapeutic agent of thrombocytopenia.

Formulation Example

The compound 1 (4g) and mannitol (50 g) were dissolved in distilled water for injection (1 liter) containing polyethylene glycol 400 (30% w/w). The solution was subjected to filtration under sterilization, and 1 ml each of which was then distributed in one ampule to prepare intravenous injection containing 4 mg of the compound 1 per ampoule.

## Claims

1. A compound of the formula:

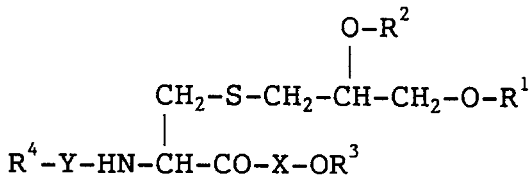

wherein each of $R^1$ and $R^2$ is hydrogen or an aliphatic acyl group, each of $R^3$ and $R^4$ is hydrogen or a protecting group, X is an amino acid sequence consisting of 1 to 10 amino acid residues which may be protected, Y is an amino acid sequence consisting of 1 to 5 amino acids residues which may be protected, bounding at a terminal carboxyl group, or a salt thereof.

2. A compound according to claim 1, wherein the aliphatic acyl group is a $C_{2-30}$ aliphatic acyl group.

3. A compound according to claim 1, wherein at least one of $R^1$ and $R^2$ is an aliphatic acyl group.

4. A compound according to any one of claims 1 to 3, wherein X is an amino acid sequence consisting of 1 to 10 amino acid residues which may be protected, containing at least an amino acid residue which have a water-solubility enhancing group.

5. A compound according to any one of claims 1 to 4, wherein the amino acid residue having a water-solubility enhancing group is an acidic amino acid residue.

6. A compound according to any one of claims 1 to 4, wherein the amino acid residue having a water-solubility enhancing group is a basic amino acid residue.

7. A compound according to claim 1, wherein at least one of $R^1$ and $R^2$ is a $C_{2-18}$ aliphatic acyl group, X is an amino acid sequence consisting of 2 to 5 optionally protected amino acid residues being selected from the group consisting of glutamyl, D-glutamyl, glycyl and lysyl, Y is an amino acid sequence consisting of 1 to 4 optionally protected amino acid residues being selected from the group consisting of glutamyl, glycyl, tyrosyl, lysyl and $\beta$-alanyl.

8. A compound according to claim 1, wherein the compound is (2R,6R.-2-glycyl-amino-6,7-bis-(hexadecanoyloxy)-4-thiaheptanoyl-glutamyl-glycyl-D-glutamic acid.

9. A compound according to claim 1, wherein the compound is (2R,6R)-2-glutamyl-glycyl-amino-6,7-bis-(hexadecanoyloxy)-4-thiaheptanoyl-glutamyl-glycyl-D-glutamic acid.

10. A compound according to claim 1, wherein the compound is (2R,6R)-2-tyrosyl-amino-6,7-bis-(hexadecanoyloxy)-4-thiaheptanoyl-glutamyl-glycyl-D-glutamic acid.

11. A compound according to claim 1, wherein the compound is (2R,6R)-2-lysyl-amino-6,7-bis-(hexadecanoyloxy)-4-thiaheptanoyl-glutamyl-glycyl-D-glutamic acid.

12. A compound according to claim 1, wherein the compound is (2R,6R)-2-glycyl-amino-6,7-bis-(hexadecanoyloxy)-4-thiaheptanoyl-glycyl-glutamyl-glutamic acid.

13. An immuno-enhancing composition having a leukocyte-increasing action, which comprises a compound or a salt thereof as defined in claim 1.

14. A composition for treating thrombocytopenia, which comprises a compound or a salt thereof as defined in claim 1.

15. Use of a compound or a salt thereof as defined in claim 1 for the preparation of a therapeutic or prophylactic agent for immuno-enhancing with a leukocyte-increasing action.

16. Use of a compound or a salt thereof as defined in claim 1 for the preparation of a therapeutic agent for thrombocytopenia.

European Patent Office

# EUROPEAN SEARCH REPORT

Application Number
EP 94 11 1469

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | INNOVATION PERSPECT. SOLID PHASE SYNTH. COLLECT.PAP., INT.SYMP., 2ND 1992 pages 433 - 436 METZGER J. ET AL. 'Solid Phase Peptide Synthesis of Lipopeptide Analogues of Bacterial Lipoprotein with Fmoc-S-(2,3-Dihydroxypropyl)- Cysteine' * figure 2 * | 1,2,4 | C07K5/06 C07K5/08 C07K5/10 C07K7/06 |
| Y | INT.J.PEPTIDE PROTEIN RES. vol. 37 , 1991 pages 46 - 57 J.METZGER ET AL. 'Synthesis of Novel Immunologically Active Tripalmityoyl-S-glycerylcysteinyl lipopeptides as Useful Intermediates for Immunogen Preparations' * the whole document * | 1-9, 11-16 | |
| Y | EP-A-0 014 815 (CIBA-GEIGY AG) 3 September 1980 * the whole document * | 1-9, 11-16 | TECHNICAL FIELDS SEARCHED (Int.Cl.6) C07K |

The present search report has been drawn up for all claims

| Place of search | Date of completion of the search | Examiner |
|---|---|---|
| MUNICH | 8 November 1994 | Deffner, C-A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)